(19) Europäisches Patentamt / European Patent Office / Office européen des brevets

(11) **EP 2 118 125 B1**

(12) **EUROPEAN PATENT SPECIFICATION**

(45) Date of publication and mention of the grant of the patent:
**26.10.2011 Bulletin 2011/43**

(51) Int Cl.:
***C07K 14/16*** (2006.01)    ***C07K 14/47*** (2006.01)
***C12N 15/62*** (2006.01)

(21) Application number: **08716417.4**

(22) Date of filing: **11.03.2008**

(86) International application number:
**PCT/EP2008/001908**

(87) International publication number:
**WO 2008/110332 (18.09.2008 Gazette 2008/38)**

(54) **PEPTIDE-COMPLEMENT CONJUGATES**

PEPTID-KOMPLEMENT-KONJUGATE

CONJUGUÉS DE COMPLÉMENT PEPTIDIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **13.03.2007 EP 07005096**

(43) Date of publication of application:
**18.11.2009 Bulletin 2009/47**

(73) Proprietor: **F. Hoffmann-La Roche AG**
**4070 Basel (CH)**

(72) Inventors:
• **DUEFEL, Hartmut**
**82444 Schlehdorf (DE)**
• **FALKENSTEIN, Roberto**
**80689 Muenchen (DE)**
• **LEIN, Iris**
**82362 Weilheim (DE)**
• **SCHMUCK, Rainer**
**83671 Benediktbeuern (DE)**
• **TISCHER, Wilhelm**
**82380 Peissenberg (DE)**

(74) Representative: **Burger, Alexander et al**
**Roche Diagnostics GmbH**
**Patent Department (TR-E)**
**Postfach 11 52**
**82372 Penzberg (DE)**

(56) References cited:
**WO-A-02/103026**

• **DATABASE UniProt human C1QA XP002433663 retrieved from EBI Database accession no. P02745**
• **THIELENS N M ET AL: "Further characterization of the interaction between the C1q subcomponent of human C1 and the transmembrane envelope glycoprotein gp41 of HIV-1" JOURNAL OF IMMUNOLOGY, THE WILLIAMS AND WILKINS CO. BALTIMORE, US, vol. 151, no. 11, 1 December 1993 (1993-12-01), pages 6583-6592, XP002117221 ISSN: 0022-1767 cited in the application**
• **ROOT MICHAEL J ET AL: "HIV-1 gp41 as a target for viral entry inhibition" CURRENT PHARMACEUTICAL DESIGN, vol. 10, no. 15, 2004, pages 1805-1825, XP009083745 ISSN: 1381-6128 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

    <110> F. Hoffmann-La Roche AG

    <120> Peptide-complement conjugates

    <130> 24171

    <150> EP 07005096.8
    <151> 2007-03-13

    <160> 52

    <170> PatentIn version 3.2

    <210> 1
    <211> 223
    <212> PRT
    <213> Homo sapiens

    <400> 1

```
Glu Asp Leu Cys Arg Ala Pro Asp Gly Lys Lys Gly Glu Ala Gly Arg
1               5               10              15

Pro Gly Arg Arg Gly Arg Pro Gly Leu Lys Gly Glu Gln Gly Glu Pro
            20              25              30

Gly Ala Pro Gly Ile Arg Thr Gly Ile Gln Gly Leu Lys Gly Asp Gln
            35              40              45

Gly Glu Pro Gly Pro Ser Gly Asn Pro Gly Lys Val Gly Tyr Pro Gly
        50              55              60

Pro Ser Gly Pro Leu Gly Ala Arg Gly Ile Pro Gly Ile Lys Gly Thr
65              70              75              80

Lys Gly Ser Pro Gly Asn Ile Lys Asp Gln Pro Arg Pro Ala Phe Ser
                85              90              95

Ala Ile Arg Arg Asn Pro Pro Met Gly Gly Asn Val Val Ile Phe Asp
            100             105             110

Thr Val Ile Thr Asn Gln Glu Glu Pro Tyr Gln Asn His Ser Gly Arg
            115             120             125

Phe Val Cys Thr Val Pro Gly Tyr Tyr Tyr Phe Thr Phe Gln Val Leu
            130             135             140

Ser Gln Trp Glu Ile Cys Leu Ser Ile Val Ser Ser Ser Arg Gly Gln
145             150             155             160

Val Arg Arg Ser Leu Gly Phe Cys Asp Thr Thr Asn Lys Gly Leu Phe
            165             170             175

Gln Val Val Ser Gly Gly Met Val Leu Gln Leu Gln Gln Gly Asp Gln
            180             185             190

Val Trp Val Glu Lys Asp Pro Lys Lys Gly His Ile Tyr Gln Gly Ser
            195             200             205

Glu Ala Asp Ser Val Phe Ser Gly Phe Leu Ile Phe Pro Ser Ala
            210             215             220
```

<210> 2
<211> 17

<212> PRT
<213> Homo sapiens

<400> 2

```
Lys Gly Ser Pro Gly Asn Ile Lys Asp Gln Pro Arg Pro Ala Phe Ser
1               5               10              15

Ala
```

<210> 3
<211> 18
<212> PRT
<213> Homo sapiens

<400> 3

```
Lys Gly Ser Pro Gly Asn Ile Lys Asp Gln Pro Arg Pro Ala Phe Ser
1               5               10              15

Ala Ile
```

<210> 4
<211> 31
<212> PRT
<213> Homo sapiens

<400> 4

```
Gly Ala Arg Gly Ile Pro Gly Ile Lys Gly Thr Lys Gly Ser Pro Gly
1               5               10              15

Asn Ile Lys Asp Gln Pro Arg Pro Ala Phe Ser Ala Ile Arg Arg
              20              25              30
```

<210> 5
<211> 154
<212> PRT
<213> Homo sapiens

<400> 5

```
Gly Ala Arg Gly Ile Pro Gly Ile Lys Gly Thr Lys Gly Ser Pro Gly
1               5                   10                  15

Asn Ile Lys Asp Gln Pro Arg Pro Ala Phe Ser Ala Ile Arg Arg Asn
                20                  25                  30

Pro Pro Met Gly Gly Asn Val Val Ile Phe Asp Thr Val Ile Thr Asn
            35                  40                  45

Gln Glu Glu Pro Tyr Gln Asn His Ser Gly Arg Phe Val Cys Thr Val
            50                  55                  60

Pro Gly Tyr Tyr Tyr Phe Thr Phe Gln Val Leu Ser Gln Trp Glu Ile
65                  70                  75                  80

Cys Leu Ser Ile Val Ser Ser Ser Arg Gly Gln Val Arg Arg Ser Leu
                85                  90                  95

Gly Phe Cys Asp Thr Thr Asn Lys Gly Leu Phe Gln Val Val Ser Gly
            100                 105                 110

Gly Met Val Leu Gln Leu Gln Gln Gly Asp Gln Val Trp Val Glu Lys
            115                 120                 125

Asp Pro Lys Lys Gly His Ile Tyr Gln Gly Ser Glu Ala Asp Ser Val
            130                 135                 140

Phe Ser Gly Phe Leu Ile Phe Pro Ser Ala
145                 150
```

<210> 6
<211> 179
<212> PRT
<213> Homo sapiens

<400> 6

```
Lys Gly Asp Gln Gly Glu Pro Gly Pro Ser Gly Asn Pro Gly Lys Val
1               5                   10                  15

Gly Tyr Pro Gly Pro Ser Gly Pro Leu Gly Ala Arg Gly Ile Pro Gly
            20                  25                  30

Ile Lys Gly Thr Lys Gly Ser Pro Gly Asn Ile Lys Asp Gln Pro Arg
            35                  40                  45

Pro Ala Phe Ser Ala Ile Arg Arg Asn Pro Pro Met Gly Gly Asn Val
        50              55                  60

Val Ile Phe Asp Thr Val Ile Thr Asn Gln Glu Glu Pro Tyr Gln Asn
65                  70                  75                  80

His Ser Gly Arg Phe Val Cys Thr Val Pro Gly Tyr Tyr Tyr Phe Thr
                85                  90                  95

Phe Gln Val Leu Ser Gln Trp Glu Ile Cys Leu Ser Ile Val Ser Ser
                100                 105                 110

Ser Arg Gly Gln Val Arg Arg Ser Leu Gly Phe Cys Asp Thr Thr Asn
            115                 120                 125

Lys Gly Leu Phe Gln Val Val Ser Gly Gly Met Val Leu Gln Leu Gln
        130             135                 140

Gln Gly Asp Gln Val Trp Val Glu Lys Asp Pro Lys Lys Gly His Ile
145                 150                 155                 160

Tyr Gln Gly Ser Glu Ala Asp Ser Val Phe Ser Gly Phe Leu Ile Phe
                165                 170                 175

Pro Ser Ala
```

```
<210> 7
<211> 345
<212> PRT
<213> Human immunodeficiency virus type 1

<220>
<221> misc_feature
<223> HIV-1 gp41 (position 507-851 of BH8 gp 160)

<400> 7
```

```
Ala Val Gly Ile Gly Ala Leu Phe Leu Gly Phe Leu Gly Ala Ala Gly
1               5                   10                  15

Ser Thr Met Gly Ala Ala Ser Met Thr Leu Thr Val Gln Ala Arg Gln
                20                  25                  30

Leu Leu Ser Gly Ile Val Gln Gln Gln Asn Asn Leu Leu Arg Ala Ile
            35                  40                  45

Glu Gly Gln Gln His Leu Leu Gln Leu Thr Val Trp Gly Ile Lys Gln
        50                  55                  60

Leu Gln Ala Arg Ile Leu Ala Val Glu Arg Tyr Leu Lys Asp Gln Gln
65                  70                  75                  80

Leu Leu Gly Ile Trp Gly Cys Ser Gly Lys Leu Ile Cys Thr Thr Ala
                85                  90                  95

Val Pro Trp Asn Ala Ser Trp Ser Asn Lys Ser Leu Glu Gln Ile Trp
                100                 105                 110

Asn Asn Met Thr Trp Met Glu Trp Asp Arg Glu Ile Asn Asn Tyr Thr
            115                 120                 125

Ser Leu Ile His Ser Leu Ile Glu Glu Ser Gln Asn Gln Gln Glu Lys
        130                 135                 140
```

```
Asn Glu Gln Glu Leu Leu Glu Leu Asp Lys Trp Ala Ser Leu Trp Asn
145             150             155                 160

Trp Phe Asn Ile Thr Asn Trp Leu Trp Tyr Ile Lys Leu Phe Ile Met
            165             170                 175

Ile Val Gly Gly Leu Val Gly Leu Arg Ile Val Phe Ala Val Leu Ser
            180             185                 190

Ile Val Asn Arg Val Arg Gln Gly Tyr Ser Pro Leu Ser Phe Gln Thr
            195             200                 205

His Leu Pro Asn Pro Arg Gly Pro Asp Arg Pro Glu Gly Ile Glu Glu
        210             215             220

Glu Gly Gly Glu Arg Asp Arg Asp Arg Ser Ile Arg Leu Val Asn Gly
225             230             235                 240

Ser Leu Ala Leu Ile Trp Asp Asp Leu Arg Ser Leu Cys Leu Phe Ser
            245             250                 255

Tyr His Arg Leu Arg Asp Leu Leu Leu Ile Val Thr Arg Ile Val Glu
            260             265                 270

Leu Leu Gly Arg Arg Gly Trp Glu Ala Leu Lys Tyr Trp Trp Asn Leu
        275             280             285

Leu Gln Tyr Trp Ser Gln Glu Leu Lys Asn Ser Ala Val Asn Leu Leu
    290             295             300

Asn Ala Thr Ala Ile Ala Val Ala Glu Gly Thr Asp Arg Val Ile Glu
305             310             315                 320

Leu Val Gln Ala Ala Tyr Arg Ala Ile Arg His Ile Pro Arg Arg Ile
            325             330                 335

Arg Gln Gly Leu Glu Arg Ile Leu Leu
        340             345
```

<210> 8
<211> 38
<212> PRT
<213> Artificial

<220>
<223> DP-107

**EP 2 118 125 B1**

<400> 8

```
Asn Asn Leu Leu Arg Ala Ile Glu Ala Gln Gln His Leu Leu Gln Leu
1               5               10              15

Thr Val Trp Gly Ile Lys Gln Leu Gln Ala Arg Ile Leu Ala Val Glu
            20              25              30

Arg Tyr Leu Lys Asp Gln
        35
```

<210> 9
<211> 38
<212> PRT
<213> Artificial

<220>
<223> DP-178

<400> 9

```
Gln Gln Glu Lys Asn Glu Gln Asp Leu Leu Ala Leu Asp Lys Trp Ala
1               5               10              15

Ser Leu Trp Thr Trp Phe Asp Ile Ser His Trp Leu Trp Tyr Ile Lys
            20              25              30

Ile Phe Ile Met Ile Val
        35
```

<210> 10
<211> 34
<212> PRT
<213> Artificial

<220>
<223> C-34

<400> 10

```
Trp Met Glu Trp Asp Arg Glu Ile Asn Asn Tyr Thr Ser Leu Ile His
1               5               10              15

Ser Leu Ile Glu Glu Ser Gln Asn Gln Gln Glu Lys Asn Glu Gln Glu
            20              25              30

Leu Leu
```

<210> 11

**26**

```
<211> 36
<212> PRT
<213> Artificial

<220>
<223> N-36

<400> 11


        Ser Gly Ile Val Gln Gln Gln Asn Asn Leu Leu Arg Ala Ile Glu Ala
        1               5               10              15


        Gln Gln His Leu Leu Gln Leu Thr Val Trp Gly Ile Lys Gln Leu Gln
                    20              25              30


        Ala Arg Ile Leu
                    35


<210> 12
<211> 36
<212> PRT
<213> Artificial

<220>
<223> T-20

<400> 12


        Tyr Thr Ser Leu Ile His Ser Leu Ile Glu Glu Ser Gln Asn Gln Gln
        1               5               10              15


        Glu Lys Asn Glu Gln Glu Leu Leu Glu Leu Asp Lys Trp Ala Ser Leu
                    20              25              30


        Trp Asn Trp Phe
                    35


<210> 13
<211> 36
<212> PRT
<213> Artificial

<220>
<223> T-651

<400> 13
```

.

```
Met Thr Trp Met Glu Trp Asp Arg Glu Ile Asn Asn Tyr Thr Ser Leu
1               5                   10                  15

Ile His Ser Leu Ile Glu Glu Ser Gln Asn Gln Gln Glu Lys Asn Glu
            20                  25                  30

Gln Glu Leu Leu
            35
```

<210> 14
<211> 39
<212> PRT
<213> Artificial

<220>
<223> T-1249

<400> 14

```
Trp Gln Glu Trp Glu Gln Lys Ile Thr Ala Leu Leu Glu Gln Ala Gln
1               5                   10                  15

Ile Gln Gln Glu Lys Asn Glu Tyr Glu Leu Gln Lys Leu Asp Lys Trp
            20                  25                  30

Ala Ser Leu Trp Glu Trp Phe
            35
```

<210> 15
<211> 39
<212> PRT
<213> Artificial

<220>
<223> T-1357

<400> 15

```
Trp Gln Glu Trp Glu Gln Lys Ile Thr Ala Leu Leu Glu Gln Ala Gln
1               5                   10                  15

Ile Gln Gln Glu Lys Asn Glu Tyr Glu Leu Gln Lys Leu Asp Lys Trp
            20                  25                  30

Ala Ser Leu Trp Glu Trp Phe
            35
```

<210> 16
<211> 58

<212> PRT
<213> Artificial

<220>
<223> T-1357 variant

<400> 16

```
Met Arg Gly Ser His His His His His His Ala Ile Asp Val Ile Glu
1               5                   10                  15

Gly Arg Trp Gln Glu Trp Glu Gln Lys Ile Thr Ala Leu Leu Glu Gln
            20                  25                  30

Ala Gln Ile Gln Gln Glu Lys Asn Glu Tyr Glu Leu Gln Lys Leu Asp
            35                  40                  45

Lys Trp Ala Ser Leu Trp Glu Trp Phe Gly
        50                  55
```

<210> 17
<211> 38
<212> PRT
<213> Artificial

<220>
<223> T-2635

<400> 17

```
Thr Thr Trp Glu Ala Trp Asp Arg Ala Ile Ala Glu Tyr Ala Ala Arg
1               5                   10                  15

Ile Glu Ala Leu Ile Arg Ala Ala Gln Glu Gln Gln Glu Lys Asn Glu
            20                  25                  30

Ala Ala Leu Arg Glu Leu
        35
```

<210> 18
<211> 123
<212> PRT
<213> Artificial

<220>
<223> HIV-1 gp41 ectodomain variant single mutant: I568P

<400> 18

```
Val Gln Ala Arg Gln Leu Leu Ser Gly Ile Val Gln Gln Gln Asn Asn
1               5               10              15


Leu Leu Arg Ala Ile Glu Gly Gln Gln His Leu Leu Gln Leu Thr Val
            20              25              30


Trp Gly Pro Lys Gln Leu Gln Ala Arg Ile Leu Ala Val Glu Arg Tyr
        35              40              45


Leu Lys Asp Gln Gln Leu Leu Gly Ile Trp Gly Cys Ser Gly Lys Leu
        50              55              60


Ile Cys Thr Thr Ala Val Pro Trp Asn Ala Ser Trp Ser Asn Lys Ser
65              70              75              80


Leu Glu Gln Ile Trp Asn Asn Met Thr Trp Met Glu Trp Asp Arg Glu
            85              90              95


Ile Asn Asn Tyr Thr Ser Leu Ile His Ser Leu Ile Glu Glu Ser Gln
            100             105             110


Asn Gln Gln Glu Lys Asn Glu Gln Glu Leu Leu
        115             120
```

<210> 19
<211> 132
<212> PRT
<213> Artificial

<220>
<223> HIV-1 gp41 ectodomain variant quadruple mutant: I568P, L550E, L566E, I580E

<400> 19

```
Met Gly Ala Ala Ser Met Thr Leu Thr Val Gln Ala Arg Gln Leu Leu
1               5                   10                  15

Ser Gly Ile Val Gln Gln Gln Asn Asn Glu Leu Arg Ala Ile Glu Gly
            20                  25                  30

Gln Gln His Leu Glu Gln Leu Thr Val Trp Gly Pro Lys Gln Leu Gln
            35                  40                  45

Ala Arg Glu Leu Ala Val Glu Arg Tyr Leu Lys Asp Gln Gln Leu Leu
        50                  55                  60

Gly Ile Trp Gly Cys Ser Gly Lys Leu Ile Cys Thr Thr Ala Val Pro
65                  70                  75                  80

Trp Asn Ala Ser Trp Ser Asn Lys Ser Leu Glu Gln Ile Trp Asn Asn
                85                  90                  95

Met Thr Trp Met Glu Trp Asp Arg Glu Ile Asn Asn Tyr Thr Ser Leu
                100                 105                 110

Ile His Ser Leu Ile Glu Glu Ser Gln Asn Gln Gln Glu Lys Asn Glu
            115                 120                 125

Gln Glu Leu Leu
        130
```

<210> 20
<211> 7
<212> PRT
<213> Artificial

<220>
<223> linker polypeptide 1

<400> 20

```
Leu Ser Leu Ser Pro Gly Lys
1               5
```

<210> 21
<211> 8
<212> PRT
<213> Artificial

<220>
<223> linker polypeptide 2

<400> 21

```
Leu Ser Pro Asn Arg Gly Glu Cys
1               5
```

<210> 22
<211> 15
<212> PRT
<213> Artificial

<220>
<223> linker polypeptide 3

<400> 22

```
Gly Gln Gln Gln Gln Gly Gln Gln Gln Gln Gly Gln Gln Gln Gln
1               5                   10                  15
```

<210> 23
<211> 16
<212> PRT
<213> Artificial

<220>
<223> linker polypeptide 4

<400> 23

```
Gly Gln Gln Gln Gln Gly Gln Gln Gln Gln Gly Gln Gln Gln Gln Gly
1               5                   10                  15
```

<210> 24
<211> 18
<212> PRT
<213> Artificial

<220>
<223> linker polypeptide 5

<400> 24

```
Gly Gln Gln Gln Gln Gly Gln Gln Gln Gln Gly Gln Gln Gln Gln Gly
1               5                   10                  15

Asn Asn
```

<210> 25
<211> 16
<212> PRT
<213> Artificial

<220>
<223> linker polypeptide 6

<400> 25

```
Gly Gly Gly Ser Gly Gly Gly Gly Ser Gly Gly Gly Gly Ser Gly Gly
1               5                   10                  15
```

<210> 26
<211> 16
<212> PRT
<213> Artificial

<220>
<223> linker polypeptide 7

<400> 26

```
Gly Gly Gly Ser Gly Gly Gly Gly Ser Gly Gly Gly Gly Ser Gly Asn
1               5                   10                  15
```

<210> 27
<211> 15
<212> PRT
<213> Artificial

<220>
<223> linker polypeptide 8

<400> 27

```
Ser Gly Gly Gly Gly Ser Gly Gly Gly Gly Ser Gly Gly Gly Gly
1               5                   10                  15
```

<210> 28
<211> 16
<212> PRT
<213> Artificial

<220>
<223> linker polypeptide 9

<400> 28

```
Ser Gly Gly Gly Gly Ser Gly Gly Gly Gly Ser Gly Gly Gly Gly Gly
1               5                   10                  15
```

<210> 29
<211> 17
<212> PRT
<213> Artificial

<220>
<223> linker polypeptide 10

<400> 29

```
Gly Ser Gly Gly Gly Gly Ser Gly Gly Gly Gly Ser Gly Gly Gly Gly
1               5                   10                  15

Thr
```

<210> 30
<211> 17
<212> PRT
<213> Artificial

<220>
<223> linker polypeptide 11

<400> 30

```
Ser Gly Gly Gly Gly Ser Gly Gly Gly Gly Ser Gly Gly Gly Gly Gly
1               5                   10                  15

Gly
```

<210> 31
<211> 18
<212> PRT
<213> Artificial

<220>
<223> linker polypeptide 12

<400> 31

```
Ser Gly Gly Gly Gly Ser Gly Gly Gly Gly Ser Gly Gly Gly Gly Gly
1               5                   10                  15

Gly Thr
```

<210> 32
<211> 18
<212> PRT
<213> Artificial

<220>
<223> linker polypeptide 13

<400> 32

```
Ser Gly Gly Gly Gly Ser Gly Gly Gly Gly Ser Gly Gly Gly Gly Gly
1               5                   10                  15

Gly Asn
```

<210> 33
<211> 18
<212> PRT
<213> Artificial

<220>
<223> linker polypeptide 14

<400> 33

```
Ser Gly Gly Gly Gly Ser Gly Gly Gly Gly Ser Gly Gly Gly Gly Gly
1               5                   10                  15

Ala Ser
```

<210> 34
<211> 25
<212> PRT
<213> Artificial

<220>
<223> linker polypeptide 15

<400> 34

```
Ser Gly Gly Gly Gly Ser Gly Gly Gly Gly Ser Gly Gly Gly Gly Ser
1               5                   10                  15

Gly Gly Gly Gly Ser Gly Gly Gly Gly
            20                  25
```

<210> 35
<211> 26
<212> PRT
<213> Artificial

<220>
<223> linker polypeptide 16

<400> 35

```
Ser Gly Gly Gly Gly Ser Gly Gly Gly Gly Ser Gly Gly Gly Gly Ser
1               5                   10                  15

Gly Gly Gly Gly Ser Gly Gly Gly Gly Gly
            20                  25
```

<210> 36
<211> 27
<212> PRT
<213> Artificial

<220>
<223> linker polypeptide 17

<400> 36

```
Ser Gly Gly Gly Gly Ser Gly Gly Gly Gly Ser Gly Gly Gly Gly Ser
1               5                   10                  15

Gly Gly Gly Gly Ser Gly Gly Gly Gly Gly Gly
            20                  25
```

<210> 37
<211> 28
<212> PRT
<213> Artificial

<220>
<223> linker polypeptide 18

<400> 37

```
Ser Gly Gly Gly Gly Ser Gly Gly Gly Gly Ser Gly Gly Gly Gly Ser
1               5                   10                  15

Gly Gly Gly Gly Ser Gly Gly Gly Gly Gly Ala Ser
            20                  25
```

<210> 38
<211> 17
<212> PRT
<213> Artificial

<220>
<223> linker polypeptide 19

<400> 38

```
Gly Ser Ser Ser Ser Ser Ser Ser Ser Ser Ser Ser Ser Ser Ser Ser
1               5                   10                  15

Gly
```

<210> 39
<211> 19
<212> PRT
<213> Artificial

<220>
<223> linker polypeptide 20

<400> 39

```
Gly Ser Ser Ser Ser Ser Ser Ser Ser Ser Ser Ser Ser Ser Ser
1               5               10              15
```

```
Gly Ala Ser
```

<210> 40
<211> 18
<212> PRT
<213> Artificial

<220>
<223> linker polypeptide 24

<400> 40

```
Gly Gly Gly Gly Ser Gly Gly Gly Gly Ser Gly Gly Gly Gly Ser Gly
1               5               10              15
```

```
Ala Ser
```

<210> 41
<211> 16
<212> PRT
<213> Artificial

<220>
<223> linker polypeptide 25

<400> 41

```
Gly Gly Gly Gly Ser Gly Gly Gly Gly Ser Gly Gly Gly Gly Ser Gly
1               5               10              15
```

<210> 42
<211> 26
<212> PRT
<213> Artificial

<220>
<223> linker polypeptide 26

<400> 42

```
Gly Gly Gly Gly Ser Gly Gly Gly Gly Ser Gly Gly Gly Gly Ser Gly
1               5               10              15
```

```
Gly Gly Gly Ser Gly Gly Gly Gly Ser Gly
        20              25
```

<210> 43
<211> 17

<212> PRT
<213> Artificial

<220>
<223> linker polypeptide 27

<400> 43

```
Gly Gly Gly Gly Ser Gly Gly Gly Gly Ser Gly Gly Gly Gly Ser Gly
1               5                   10                  15

Gly
```

<210> 44
<211> 27
<212> PRT
<213> Artificial

<220>
<223> linker polypeptide 28

<400> 44

```
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly
            20                  25
```

<210> 45
<211> 6
<212> PRT
<213> Artificial

<220>
<223> linker polypeptide 29

<400> 45

```
Leu Ser Leu Ser Gly Gly
1               5
```

<210> 46
<211> 7
<212> PRT
<213> Artificial

<220>
<223> linker polypeptide 30

<400> 46

```
Leu Ser Leu Ser Pro Gly Gly
1               5
```

<210> 47
<211> 20
<212> PRT
<213> Artificial

<220>
<223> Linker polypeptide 31

<400> 47

```
Gly Gly Gly Ser Gly Gly Gly Ser Gly Gly Gly Ser Gly Gly Gly Ser
1               5               10              15

Gly Gly Gly Ser
        20
```

<210> 48
<211> 23
<212> PRT
<213> Artificial

<220>
<223> Linker polypeptide 32

<400> 48

```
Gly Gly Gly Ser Gly Gly Gly Ser Gly Gly Gly Ser Gly Gly Gly Ser
1               5               10              15

Gly Gly Gly Ser Gly Gly Gly
        20
```

<210> 49
<211> 237
<212> PRT
<213> Artificial

<220>
<223> T-1357 - ClqA

<400> 49

```
Met Arg Gly Ser His His His His His His Ala Ile Asp Val Ile Glu
1               5                   10                  15


Gly Arg Trp Gln Glu Trp Glu Gln Lys Ile Thr Ala Leu Leu Glu Gln
            20                  25                  30


Ala Gln Ile Gln Gln Glu Lys Asn Glu Tyr Glu Leu Gln Lys Leu Asp
            35                  40                  45


Lys Trp Ala Ser Leu Trp Glu Trp Phe Gly Lys Gly Asp Gln Gly Glu
            50                  55                  60


Pro Gly Pro Ser Gly Asn Pro Gly Lys Val Gly Tyr Pro Gly Pro Ser
65                  70                  75                  80


Gly Pro Leu Gly Ala Arg Gly Ile Pro Gly Ile Lys Gly Thr Lys Gly
                85                  90                  95


Ser Pro Gly Asn Ile Lys Asp Gln Pro Arg Pro Ala Phe Ser Ala Ile
            100                 105                 110


Arg Arg Asn Pro Pro Met Gly Gly Asn Val Val Ile Phe Asp Thr Val
            115                 120                 125


Ile Thr Asn Gln Glu Glu Pro Tyr Gln Asn His Ser Gly Arg Phe Val
    130                 135                 140


Cys Thr Val Pro Gly Tyr Tyr Tyr Phe Thr Phe Gln Val Leu Ser Gln
145                 150                 155                 160


Trp Glu Ile Cys Leu Ser Ile Val Ser Ser Arg Gly Gln Val Arg
                165                 170                 175


Arg Ser Leu Gly Phe Cys Asp Thr Thr Asn Lys Gly Leu Phe Gln Val
            180                 185                 190


Val Ser Gly Gly Met Val Leu Gln Leu Gln Gln Gly Asp Gln Val Trp
            195                 200                 205


Val Glu Lys Asp Pro Lys Lys Gly His Ile Tyr Gln Gly Ser Glu Ala
    210                 215                 220


Asp Ser Val Phe Ser Gly Phe Leu Ile Phe Pro Ser Ala
225                 230                 235
```

<210> 50
<211> 233
<212> PRT
<213> Artificial

<220>
<223> ROB I

<400> 50

```
Met Arg Gly Ser His His His His His His Ile Glu Gly Arg Trp Gln
1               5                   10                  15

Glu Trp Glu Gln Lys Ile Thr Ala Leu Leu Glu Gln Ala Gln Ile Gln
            20                  25                  30

Gln Glu Lys Asn Glu Tyr Glu Leu Gln Lys Leu Asp Lys Trp Ala Ser
            35                  40                  45
```

```
Leu Trp Glu Trp Phe Gly Lys Gly Asp Gln Gly Glu Pro Gly Pro Ser
    50              55              60

Gly Asn Pro Gly Lys Val Gly Tyr Pro Gly Pro Ser Gly Pro Leu Gly
65              70              75              80

Ala Arg Gly Ile Pro Gly Ile Lys Gly Thr Lys Gly Ser Pro Gly Asn
            85              90              95

Ile Lys Asp Gln Pro Arg Pro Ala Phe Ser Ala Ile Arg Arg Asn Pro
            100             105             110

Pro Met Gly Gly Asn Val Val Ile Phe Asp Thr Val Ile Thr Asn Gln
        115             120             125

Glu Glu Pro Tyr Gln Asn His Ser Gly Arg Phe Val Cys Thr Val Pro
    130             135             140

Gly Tyr Tyr Tyr Phe Thr Phe Gln Val Leu Ser Gln Trp Glu Ile Cys
145             150             155             160

Leu Ser Ile Val Ser Ser Ser Arg Gly Gln Val Arg Arg Ser Leu Gly
            165             170             175

Phe Cys Asp Thr Thr Asn Lys Gly Leu Phe Gln Val Val Ser Gly Gly
            180             185             190

Met Val Leu Gln Leu Gln Gln Gly Asp Gln Val Trp Val Glu Lys Asp
        195             200             205

Pro Lys Lys Gly His Ile Tyr Gln Gly Ser Glu Ala Asp Ser Val Phe
    210             215             220

Ser Gly Phe Leu Ile Phe Pro Ser Ala
225             230
```

<210> 51
<211> 231
<212> PRT
<213> Artificial

<220>
<223> ROB II

<400> 51

```
Met Arg Gly Ser His His His His His His Ile Glu Gly Arg Gly Ala
1           5                   10                  15


Arg Gly Ile Pro Gly Ile Lys Gly Thr Lys Gly Ser Pro Gly Asn Ile
            20                  25                  30


Lys Asp Gln Pro Arg Pro Ala Phe Ser Ala Ile Arg Arg Asn Pro Pro
            35                  40                  45


Met Gly Gly Asn Val Val Ile Phe Asp Thr Val Ile Thr Asn Gln Glu
    50                  55                  60


Glu Pro Tyr Gln Asn His Ser Gly Arg Phe Val Cys Thr Val Pro Gly
65                  70                  75                  80


Tyr Tyr Tyr Phe Thr Phe Gln Val Leu Ser Gln Trp Glu Ile Cys Leu
                85                  90                  95


Ser Ile Val Ser Ser Ser Arg Gly Gln Val Arg Arg Ser Leu Gly Phe
                100                 105                 110


Cys Asp Thr Thr Asn Lys Gly Leu Phe Gln Val Val Ser Gly Gly Met
    115                 120                 125


Val Leu Gln Leu Gln Gln Gly Asp Gln Val Trp Val Glu Lys Asp Pro
    130                 135                 140


Lys Lys Gly His Ile Tyr Gln Gly Ser Glu Ala Asp Ser Val Phe Ser
145                 150                 155                 160


Gly Phe Leu Ile Phe Pro Ser Ala Gly Gly Gly Ser Gly Gly Gly Ser
                165                 170                 175


Gly Gly Gly Ser Gly Gly Gly Ser Gly Gly Gly Ser Gly Gly Gly Trp
                180                 185                 190


Gln Glu Trp Glu Gln Lys Ile Thr Ala Leu Leu Glu Gln Ala Gln Ile
            195                 200                 205


Gln Gln Glu Lys Asn Glu Tyr Glu Leu Gln Lys Leu Asp Lys Trp Ala
    210                 215                 220


Ser Leu Trp Glu Trp Phe Gly
225                 230
```

<210> 52
<211> 51
<212> PRT
<213> Artificial

<220>
<223> T-2324

<400> 52

```
Gln Ala Arg Gln Leu Leu Ser Gly Ile Val Gln Gln Gln Asn Asn Leu
1               5               10              15

Leu Arg Ala Ile Glu Ala Gln Gln His Leu Leu Gln Leu Thr Val Trp
            20              25              30

Gly Ile Lys Gln Leu Gln Ala Arg Ile Leu Ala Val Glu Arg Tyr Leu
        35              40              45

Lys Asp Gln
        50
```

## Description

The current invention reports conjugates of antifusogenic peptides and complement factor C1q globular head derived polypeptides.

## Background of the Invention

The infection of cells by the HIV virus is effected by a process, in which the membrane of the cells to be infected and the viral membrane are fused. A general scheme for this process is proposed: The viral envelope glycoprotein complex (gp120/gp41) interacts with a cell surface receptor located on the membrane of the cell to be infected. The binding of gp120 to e.g. the CD4 receptor in combination with a co-receptor such as CCR-5 or CXCR-4, causes a change in the conformation of the gp120/gp41 complex. In consequence of this conformational change the gp41 protein is able to insert into the membrane of the target cell. This insertion is the beginning of the membrane fusion process.

It is known that the amino acid sequence of the gp41 protein differs between the different HIV strains because of naturally occurring polymorphisms. But the same domain architecture can be recognized, precisely, a fusion signal, two heptad repeat domains (HR1, HR2) and a transmembrane domain (in N- to C-terminal direction). It is suggested that the fusion (or fusogenic) domain is participating in the insertion into and disintegration of the cell membrane. The HR regions are built up of multiple stretches each comprising 7 amino acids ("heptad") (see e.g. Shu, W., et al., Biochemistry 38 (1999) 5378-5385). Beside the heptads one or more leucine zipper-like motifs are present. This composition accounts for the formation of a coiled coil structure of the gp41 protein and just as well of peptide derived from these domains. Coiled coils are in general oligomers consisting of two or more interacting helices.

Peptides with amino acid sequences deduced from the HR1 or HR2 domain of gp41 are effective in vitro and in vivo inhibitors of HIV uptake into cells (for example peptides see e.g. US 5,464,933, US 5,656,480, US 6,258,782, US 6,348,568, or US 6,656,906). For example, T20 (also known as DP178, Fuzeon®, a HR2 peptide) and T651 (US 6,479,055) are very potent inhibitors of HIV infection. It has been attempted to enhance the efficacy of HR2 derived peptides, with for example amino acid substitutions or chemical crosslinking (Sia, S.K., et al, Proc. Natl. Acad. Sci. USA 99 (2002) 14664-14669; Otaka, A., et al, Angew. Chem. Int. Ed. 41 (2002) 2937-2940).

The inborn immunity of humans comprises the complement pathway. This pathway is activated by the binding of C1q, the recognition subunit of the C1 complement factor, to an immunological target. The full C1q molecule is a heteromeric molecule comprising six copies of each of the three monomeric building blocks, which are denoted as C1qA, C1qB, and C1qC. Each of the monomeric units comprises an N-terminal region (of 3 to 9 residues), a collagen-like domain (spanning approximately 81 residues), and a globular domain (globular head; spanning approximately 135 residues) (Sellar, G.C., et al. Biochem. J. 274 (1991) 481-490).

Moir et al. report the HIV-1 infection of B-cells via the activation of the complement pathway (Moir, S., et al., J. Exp. Med. 192 (2000) 637-646). The activation of the human complement pathway is emerging from a binding of the immunodominant region of gp41 (position 590-620 of gp160; numbering according to Ratner, L., et al., Nature 313 (1985) 277-284) with complement factor C1q (Ebenbichler, C.F., J. Exp. Med. 174 (1991) 1417-1424). Thielens et al. report that the C1q subcomponent of human C1 and the transmembrane envelope glycoprotein gp41 of HIV-1 interact in the region of amino acid positions 590-613 of gp160. This interaction can be inhibited by a peptide comprising the amino acids 601-613 of gp160 with a disulphide bond connecting Cys 605 and Cys 611 (Thielens, N.M., et al., J. Immunol. 151 (1993) 6583-6592). In WO 02/103026 a method for the recombinant production of peptidic antiviral fusion inhibitors, and acetylation of gp41 fragments are reported. HIV-1 gp41 as a target for viral entry inhibition is reported by Root et al. (Root, M.J., et al., Curr. Pharm. Des. 10 (2004) 1805-1825).

## Summary of the Invention

The current invention comprises a polypeptide conjugate comprising a first polypeptide selected from SEQ ID NO: 01, and a second polypeptide selected from the group of linear antifusogenic peptides.

In one embodiment the conjugate according to the invention comprises the first and second polypeptide in an order selected from the following group of orders:

N-terminus - first polypeptide - second polypeptide - C-terminus,
N-terminus - second polypeptide - first polypeptide - C-terminus.

In another embodiment the conjugate according to the invention comprises between the first and the second polypeptide a linker polypeptide.

The invention further reports a polypeptide conjugate comprising

a) a first polypeptide (1st pp) selected from SEQ ID NO: 01,
b) a second polypeptide (2nd pp) selected from the group of linear antifusogenic peptides,
c) a third polypeptide (3rd pp) selected from the group of antigen-binding anti-CCR5 antibody chains, antigen-binding anti-CD4 antibody chains, neutralizing anti-HIV-1 antibody chains, and fragments thereof,
d) a linker polypeptide (link pp) connecting said first, second, and/or third polypeptide.

In this polypeptide conjugate the constituting polypeptides have an N- to C-terminal order of

$$[\text{1st pp}]_a - [\text{link pp}]_m - [\text{2nd pp}]_b - [\text{link pp}]_n - [\text{3rd pp}]_c - [\text{link pp}]_o -$$
$$[\text{1st pp}]_d - [\text{link pp}]_p - [\text{2nd pp}]_e - [\text{link pp}]_q - [\text{3rd pp}]_f - [\text{link pp}]_r -$$
$$[\text{1st pp}]_g,$$

wherein a, b, c, d, e, f, g, m, n, o, p, q, r are all an integer of 0 or 1, and with

a+d+g=1,
b+e=1,
c+f=0 or 1,
m, n, o, p, q, r are independently of each other either 0 or 1.

A value of 0 denotes the absence of the corresponding polypeptide at the corresponding position, and a value of 1 denotes the presence of the corresponding polypeptide at the corresponding position in the polypeptide conjugate.
In one embodiment the optional third polypeptide is selected from the group of antigen-binding anti-CCR5 antibody chains and fragments thereof.
In another embodiment is the optional third polypeptide selected from antigen-binding anti-CD4 antibody chains and fragments thereof.
In another anti-HIV-1 embodiment is the optional third polypeptide selected from antigen-binding anti-HIV-1 antibody chains and fragments thereof.
In one embodiment is the linker polypeptide selected from the group of polypeptide comprising SEQ ID NO: 20 to SEQ ID NO: 48.
In another embodiment is the second polypeptide selected from the group of antifusogenic peptides comprising SEQ ID NO: 08 to SEQ ID NO: 19.
Another aspects of the current invention are a nucleic acid encoding a polypeptide conjugate according to the invention and a eukaryotic cell comprising the nucleic acid according to the invention.
The invention further comprises a method for the production of a polypeptide conjugate according to the invention comprising the following steps:

a) cultivating a cell comprising a nucleic acid encoding a polypeptide conjugate according to the invention under conditions suitable for the expression of the polypeptide conjugate, and
b) recovering the polypeptide conjugate from the cell or the culture medium.

The invention also comprises a pharmaceutical composition, containing a polypeptide conjugate according to the invention or a pharmaceutically acceptable salt thereof together with a pharmaceutically acceptable excipient or carrier.
Also encompassed by the current invention is the use of a polypeptide conjugate according to the invention for the manufacture of a medicament for the treatment of viral infections, preferably of a HIV infection.

## Detailed Description of the Invention

The current invention comprises a polypeptide conjugate comprising a first polypeptide selected from SEQ ID NO: 01, and a second polypeptide selected from the group of linear antifusogenic peptides. The invention further comprises a polypeptide conjugate comprising a first polypeptide (1st pp) selected from SEQ ID NO: 01, and a second polypeptide (2nd pp) selected from the group of linear antifusogenic peptides, and a third polypeptide (3rd pp) selected from the group comprising antigen-binding fragments of anti-CCR5 antibodies, neutralizing fragments of anti-HIV-1 antibodies, and antigen-binding fragments of anti-CD4 antibodies, and a linker polypeptide (link pp) connecting said first, second, and/or third polypeptide, whereby in this polypeptide conjugate the individual polypeptides have an N- to C-terminal order of

$$[1\text{st pp}]_a - [\text{link pp}]_m - [2\text{nd pp}]_b - [\text{link pp}]_n - [3\text{rd pp}]_c - [\text{link pp}]_o -$$
$$[1\text{st pp}]_d - [\text{link pp}]_p - [2\text{nd pp}]_e - [\text{link pp}]_q - [3\text{rd pp}]_f - [\text{link pp}]_r -$$
$$[1\text{st pp}]_g$$

wherein a, b, c, d, e, f, g, m, n, o, p, q, r are all an integer of from 0 to 1, and with the proviso that

a+d+g=1,

b+e=1,

c+f=0 or 1,

m, n, o, p, q, r are independently of each other either 0 or 1,

wherein a value of 0 denotes the absence of the corresponding polypeptide at the corresponding position, and a value of 1 denotes the presence of the corresponding polypeptide at the corresponding position in the polypeptide conjugate.

Methods and techniques useful for carrying out the current invention are known to a person skilled in the art and are described e.g. in Thielens, N.M., et al., J. Immunol. 151 (1993) 6583-6592; Ausubel, F.M., ed., Current Protocols in Molecular Biology, Volumes I to III (1997), and Sambrook et al., Molecular Cloning: A Laboratory Manual, Second Edition, Cold Spring Harbor Laboratory Press, Cold Spring Harbor, N.Y. (1989). As known to a person skilled in the art enables the use of recombinant DNA technology the production of numerous derivatives of a nucleic acid and/or polypeptide. Such derivatives can, for example, be modified in one individual or several positions by substitution, alteration, exchange, deletion, or insertion. The modification or derivatisation can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA). The use of recombinant technology enables a person skilled in the art to transform various host cells with one or more heterologous nucleic acids. Although the transcription and translation, i.e. expression, machinery of different cells use the same elements, cells belonging to different species may have among other things a different so-called codon usage. Thereby identical polypeptides (with respect to amino acid sequence) may be encoded by different nucleic acid(s). Also, due to the degeneracy of the genetic code, different nucleic acids may encode the same polypeptide.

A "nucleic acid" as used herein, refers to a polynucleotide molecule consisting of individual nucleotides, for example to DNA, RNA, or modifications thereof. This polynucleotide molecule can be a naturally occurring polynucleotide molecule or a synthetic polynucleotide molecule or a combination of one or more naturally occurring polynucleotide molecules with one or more synthetic polynucleotide molecules. Also encompassed by this definition are naturally occurring polynucleotide molecules in which one or more nucleotides are changed, e.g. by mutagenesis, deleted, or added. A nucleic acid can either be isolated, or integrated in another nucleic acid, e.g. in an expression cassette, a plasmid, or the genome of a host cell. A nucleic acid is characterized by its nucleic acid sequence consisting of individual nucleotides. To a person skilled in the art procedures and methods are well known to convert an amino acid sequence of, e.g., a polypeptide to a corresponding nucleic acid sequence encoding this amino acid sequence. Therefore, a nucleic acid is characterized by its nucleic acid sequence consisting of individual nucleotides and likewise by the amino acid sequence of a polypeptide encoded thereby.

A "nucleic acid" as used herein, refers to a naturally occurring or partially or fully non-naturally occurring nucleic acid encoding a polypeptide which can be produced recombinantly. The nucleic acid can be build up of DNA-fragments which are either isolated or synthesized by chemical means. The nucleic acid can be integrated into another nucleic acid, e.g. in an expression plasmid or the genome/chromosome of a eukaryotic host cell. Plasmid includes shuttle and expression plasmids. Typically, the plasmid will also comprise a prokaryotic propagation unit comprising an origin of replication (e.g. the ColE1 origin of replication) and a selectable marker (e.g. ampicillin or tetracycline resistance gene), for replication and selection, respectively, of the plasmid in prokaryotes.

An "expression cassette" refers to a nucleic acid that contains the elements necessary for expression and secretion of at least the contained structural gene in a cell.

A "gene" denotes a segment e.g. on a chromosome or on a plasmid which is necessary for the expression of a peptide, polypeptide, or protein. Beside the coding region the gene comprises other functional elements including a promoter, introns, and terminators.

A "structural gene" denotes the coding region of a gene without a signal sequence.

A "selectable marker" is a gene that allows cells carrying the gene to be specifically selected for or against, in the presence of a corresponding "selection agent". A useful positive selectable marker is an antibiotic resistance gene. This selectable marker allows the host cell transformed with the gene to be positively selected for in the presence of the corresponding antibiotic, e.g. selection agent; a non-transformed host cell will not be capable to grow or survive under the selective culture conditions in the presence of the selection agent. Selectable markers can be positive, negative, or bifunctional. Positive selectable markers allow selection for cells carrying the marker, whereas negative selectable markers allow cells carrying the marker to be selectively eliminated. Typically, a selectable marker will confer resistance to a drug or compensate for a metabolic or catabolic defect in the host cell. Selectable markers useful with eukaryotic cells include, e.g., the genes for aminoglycoside phosphotransferase (APH), such as the hygromycin phosphotransferase (hyg), neomycin and G418 APH, dihydrofolate reductase (DHFR), thymidine kinase (tk), glutamine synthetase (GS), asparagine synthetase, tryptophan synthetase (indole), histidinol dehydrogenase (histidinol D), and genes providing resistance to puromycin, bleomycin, phleomycin, chloramphenicol, Zeocin, and mycophenolic acid. Further selectable marker genes are described in WO 92/08796 and WO 94/28143.

"Regulatory elements" as used herein, refer to nucleotide sequences present in cis, necessary for transcription and/or translation of the gene comprising the nucleic acid encoding a polypeptide of interest. The transcriptional regulatory elements normally comprise a promoter upstream of the structural gene to be expressed, transcriptional initiation and termination sites, and a polyadenylation signal sequence. The term "transcriptional initiation site" refers to the nucleotide, or more precisely to the nucleic acid base, in the gene corresponding to the first nucleotide incorporated into the primary transcript, i.e. the mRNA precursor; the transcriptional initiation site may overlap with the promoter sequence. The term "transcriptional termination site" refers to a nucleotide sequence normally present at the 3' end of a gene of interest to be transcribed, that causes RNA polymerase to terminate transcription. The polyadenylation signal nucleotide sequence, or poly-A addition signal provides the signal for the cleavage at a specific site at the 3' end of eukaryotic mRNA and the post-transcriptional addition in the nucleus of a sequence of about 100-200 adenine nucleotides (polyA tail) to the cleaved 3' end. The polyadenylation signal sequence may include the consensus sequence AATAAA located at about 10-30 nucleotides upstream from the site of cleavage.

To produce a secreted polypeptide, the structural gene of interest includes a DNA segment that encodes a "signal sequence" or "leader peptide". The signal sequence directs the newly synthesized polypeptide to and through the membrane of the Endoplasmatic Reticulum (ER) where the polypeptide can be routed for secretion. The signal sequence is cleaved off by a signal peptidases during the protein crosses the ER membrane. As for the function of the signal sequence the recognition by the host cell's secretion machinery is essential. Therefore the used signal sequence has to be recognized by the host cell's proteins and enzymes of the secretion machinery.

Translational regulatory elements include a translational initiation (AUG) and translation stop codon (TAA, TAG or TGA). An internal ribosome entry site (IRES) can be included in some constructs.

A "promoter" refers to a polynucleotide sequence that controls transcription of a gene/structural gene or nucleic acid sequence to which it is operably linked. A promoter includes signals for RNA polymerase binding and transcription initiation. The promoters used will be functional in the cell type of the host cell in which expression of the selected nucleic acid sequence is contemplated. A large number of promoters including constitutive, inducible, and repressible promoters from a variety of different sources, are well known in the art (and identified in databases such as GenBank) and are available as or within cloned polynucleotides (from, e.g., depositories such as ATCC as well as other commercial or individual sources). A "promoter" comprises a nucleotide sequence that directs the transcription of a structural gene. Typically, a promoter is located in the 5' noncoding or untranslated region of a gene, proximal to the transcriptional start site of a structural gene. Sequence elements within promoters that function in the initiation of transcription are often characterized by consensus nucleotide sequences. These promoter elements include RNA polymerase binding sites, TATA sequences, CAAT sequences, differentiation-specific elements (DSEs; McGehee, R.E., et al., Mol. Endocrinol. 7 (1993) 551-560), cyclic AMP response elements (CREs), serum response elements (SREs; Treisman, R., Seminars in Cancer Biol. 1 (1990) 47-58), glucocorticoid response elements (GREs), and binding sites for other transcription factors, such as CRE/ATF (O'Reilly, M.A., et al., J. Biol. Chem. 267 (1992) 19938-19943), AP2 (Ye, J., et al., J. Biol. Chem. 269 (1994) 25728), SP1, cAMP response element binding protein (CREB; Loeken, M.R., Gene Expr. 3 (1993) 253-264), and octamer factors (see, in general, Watson et al., eds., Molecular Biology of the Gene, 4th ed. (The Benjamin/ Cummings Publishing Company, Inc. 1987), and Lemaigre, F.P. and Rousseau, G.G., Biochem. J. 303 (1994) 1-14). If a promoter is an inducible promoter, then the rate of transcription increases in response to an inducing agent. In contrast, the rate of transcription is not regulated by an inducing agent if the promoter is a constitutive promoter. Repressible promoters are also known. For example, the c-fos promoter is specifically activated upon binding of growth hormone to its receptor on the cell surface. Tetracycline (tet) regulated expression can be achieved by artificial hybrid promoters that consist e.g. of a CMV promoter followed by two Tet-operator sites. The Tet-repressor binds to the two Tet-operator sites and blocks transcription. Upon addition of the inducer tetracycline, Tet-repressor is released from the Tet-operator sites and transcription proceeds (Gossen, M. and Bujard, H. PNAS 89 (1992) 5547-5551). For other inducible promoters including metallothionein and heat shock promoters, see, e.g., Sambrook et al. (supra) and Gossen, M., et al., Curr. Opin. Biotech. 5 (1994) 516-520. Among the eukaryotic promoters that have been identified as strong promoters for high-level expression are the SV40 early promoter, adenovirus major late promoter, mouse metallothionein-I promoter, Rous sarcoma virus long terminal repeat, Chinese hamster elongation factor 1 alpha (CHEF-1, see e.g. US 5,888,809), human EF-1 alpha, ubiquitin, and human cytomegalovirus immediate early promoter (CMV IE). The "promoter" can be constitutive or inducible. An enhancer (i.e., a cis-acting DNA element that acts on a promoter to increase transcription) may be necessary to function in conjunction with the promoter to increase the level of expression obtained with a promoter alone, and may be included as a transcriptional regulatory element. Often, the polynucleotide segment containing the promoter will include enhancer sequences as well (e.g., CMV or SV40).

An "enhancer", as used herein, refers to a polynucleotide sequence that enhances transcription of a gene or coding sequence to which it is operably linked. Unlike promoters, enhancers are relatively orientation and position independent and have been found 5' or 3' (Lusky, M., et al., Mol. Cell Bio., 3 (1983) 1108-1122) to the transcription unit, within an intron (Banerji, J., et al., Cell, 33 (1983) 729-740) as well as within the coding sequence itself (Osborne, T.F., et al., Mol. Cell Bio., 4 (1984) 1293-1305). Therefore, enhancers may be placed upstream or downstream from the transcription initiation site or at considerable distances from the promoter, although in practice enhancers may overlap physically and functionally with promoters. A large number of enhancers, from a variety of different sources are well known in the art (and identified in databases such as GenBank) and available as or within cloned polynucleotide sequences (from, e.g., depositories such as the ATCC as well as other commercial or individual sources). A number of polynucleotides comprising promoter sequences (such as the commonly-used CMV promoter) also comprise enhancer sequences. For example, all of the strong promoters listed above may also contain strong enhancers (see e.g. Bendig, M.M., Genetic Engineering, 7 (Academic Press, 1988) 91-127).

An "internal ribosome entry site" or "IRES" describes a sequence which functionally promotes translation initiation independent from the gene 5' of the IRES and allows two cistrons (open reading frames) to be translated from a single transcript in an animal cell. The IRES provides an independent ribosome entry site for translation of the open reading frame immediately downstream (downstream is used interchangeably herein with 3') of it. Unlike bacterial mRNA which can be polycistronic, i.e. encode several different polypeptides that are translated sequentially from the mRNAs, most mRNAs of animal cells are monocistronic and code for the synthesis of only one polypeptide or protein. With a polycistronic transcript in a eukaryotic cell, translation would initiate from the most 5' translation initiation site, terminate at the first stop codon, and the transcript would be released from the ribosome, resulting in the translation of only the first encoded polypeptide in the mRNA. In a eukaryotic cell, a polycistronic transcript having an IRES operably linked to the second or subsequent open reading frame in the transcript allows the sequential translation of that downstream open reading frame to produce the two or more polypeptides encoded by the same transcript. The use of IRES elements in vector construction has been previously described, see, e.g., Pelletier, J., et al., Nature 334 (1988) 320-325; Jang, S.K., et al., J. Virol. 63 (1989) 1651- 1660; Davies, M.V., et al., J. Virol. 66 (1992) 1924-1932; Adam, M.A., et al. J. Virol. 65 (1991) 4985- 4990; Morgan, R.A., et al. Nucl. Acids Res. 20 (1992) 1293-1299; Sugimoto, Y, et al. Biotechnology 12 (1994) 694-698; Ramesh, N., et al. Nucl. Acids Res. 24 (1996) 2697-2700; and Mosser, D.D. et al, BioTechniques 22 (1997) 150-161).

"Operably linked" refers to a juxtaposition of two or more components, wherein the components so described are in a relationship permitting them to function in their intended manner. For example, a promoter and/or enhancer are operably linked to a coding sequence, if it acts in cis to control or modulate the transcription of the linked sequence. Generally, but not necessarily, the DNA sequences that are "operably linked" are contiguous and, where necessary to join two protein encoding regions such as a secretory leader and a polypeptide, or two polypeptides, contiguous and in reading frame. However, although an operably linked promoter is generally located upstream of the coding sequence, it is not necessarily contiguous with it. Enhancers do not have to be contiguous. An enhancer is operably linked to a coding sequence if the enhancer increases transcription of the coding sequence. Operably linked enhancers can be located upstream, within, or downstream of coding sequences and at considerable distance from the promoter. A polyadenylation site is operably linked to a coding sequence if it is located at the downstream end (3' end) of the coding sequence such that transcription proceeds through the coding sequence into the polyadenylation sequence. Linking is accomplished by recombinant methods known in the art, e.g., using PCR methodology and/or by ligation at convenient restriction sites. If convenient restriction sites do not exist, then synthetic oligonucleotide adaptors or linkers are used in accord with conventional practice.

The term "expression" as used herein refers to transcription and/or translation occurring within a cell, e.g. a host cell producing a recombinant polypeptide. The level of transcription of a desired product in a host cell can be determined on the basis of the amount of corresponding mRNA that is present in the cell. For example, mRNA transcribed from a sequence can be quantitated by PCR or by Northern hybridization (see Sambrook et al., Molecular Cloning: A Laboratory Manual, Cold Spring Harbor Laboratory Press (1989)). Protein encoded by a nucleic acid or structural gene can be quantitated by various methods, e.g. by ELISA, by assaying for the biological activity of the protein, or by employing assays that are independent of such activity, such as Western blotting or radioimmunoassay, using antibodies that recognize and bind to the protein (see Sambrook et al., 1989, supra).

A "host cell" refers to a cell into which a nucleic acid to be expressed or to be amplified is introduced. The term "host cell" includes both prokaryotic cells, which are used for propagation of plasmids, and eukaryotic cells, which are used for the expression of a nucleic acid. Preferably, the prokaryotic cells are E.coli cells. Preferably, the eukaryotic cells are mammalian cells. Preferably the mammalian cell is selected from the group of mammalian cells comprising CHO cells (e.g. CHO K1, CHO DG44), BHK cells, NS0 cells, SP2/0 cells, HEK 293 cells, HEK 293 EBNA cells, PER.C6® cells, and COS cells. As used herein, the expression "cell" includes the subject cell and its progeny. Thus, the words "transformant", "transformed cell", "transfectant", and "transfected cell" include the primary subject cell and cultures derived there from without regard for the number of transfers. It is also understood that all progeny may not be precisely identical in DNA content, due to deliberate or inadvertent mutations. Variant progeny that have the same function or biological activity as screened for in the originally transformed cell are included.

A "polypeptide" is a polymer of amino acid residues joined by peptide bonds, whether produced naturally or synthetically. Polypeptides of less than about 20 amino acid residues may be referred to as "peptides." A "protein" is a polypeptide comprising one or more polypeptide chains whereby at least one chain has a length of 100 amino acids or more. A protein may also comprise non-peptidic components, such as carbohydrate groups. Carbohydrates and other non-peptidic substituents may be added to a protein by the cell in which the protein is produced, and may vary with the type of cell. Proteins are defined herein in terms of their amino acid backbone structures; additions such as carbohydrate groups are generally not specified, but may be present nonetheless.

"Heterologous DNA" or "heterologous polypeptide" refers to a DNA molecule or a polypeptide, or a population of DNA molecules or a population of polypeptides, that do not exist naturally within a given host cell. DNA molecules heterologous to a particular host cell may contain DNA derived from the host cell species (i.e. endogenous DNA) so long as that host DNA is combined with non-host DNA (i.e. exogenous DNA). For example, a DNA molecule containing a non-host DNA segment encoding a polypeptide operably linked to a host DNA segment comprising a promoter is considered to be a heterologous DNA molecule. Conversely, a heterologous DNA molecule can comprise an endogenous structural gene operably linked with an exogenous promoter. A polypeptide encoded by a non-host DNA molecule is a "heterologous" polypeptide.

A "cloning plasmid" is a nucleic acid, such as a plasmid, cosmid, phageimid, or bacterial artificial chromosome (BAC), which has the capability of replicating autonomously in a host cell. Cloning plasmids typically contain one or a small number of restriction endonuclease recognition sites that allow insertion of a nucleic acid in a determinable fashion without loss of an essential biological function of the plasmid, as well as nucleotide sequences encoding a selectable marker that is suitable for use in the identification and selection of cells transformed with the cloning plasmid. Resistance genes typically include genes that provide tetracycline, ampicillin, or neomycin resistance.

An "expression plasmid" is a nucleic acid encoding a polypeptide to be expressed in a host cell. Typically, an expression plasmid comprises a prokaryotic plasmid propagation unit, e.g. for E.coli, comprising an origin of replication, and a selection marker, an eukaryotic selection marker, and one or more expression cassettes for the expression of the structural gene(s) of interest each comprising a promoter, a structural gene, and a transcription terminator including a polyadenylation signal. Gene expression is usually placed under the control of a promoter, and such a structural gene is said to be "operably linked to" the promoter. Similarly, a regulatory element and a core promoter are operably linked, if the regulatory element modulates the activity of the core promoter.

An "isolated polypeptide" is a polypeptide that is essentially free from contaminating cellular components, such as carbohydrate, lipid, or other proteinaceous impurities associated with the polypeptide in nature. Typically, a preparation of isolated polypeptide contains the polypeptide in a highly purified form, i.e. at least about 80% pure, at least about 90% pure, at least about 95% pure, greater than 95% pure, or greater than 99% pure. One way to show that a particular protein preparation contains an isolated polypeptide is by the appearance of a single band following sodium dodecyl sulfate (SDS)-polyacrylamide gel electrophoresis of the protein preparation and Coomassie Brilliant Blue staining of the gel. However, the term "isolated" does not exclude the presence of the same polypeptide in alternative physical forms, such as dimers or alternatively glycosylated or derivatized forms.

The term "immunoglobulin" refers to a protein consisting of one or more polypeptides substantially encoded by an immunoglobulin gene. The different polypeptides of which an immunoglobulin is composed of are referred to depending on their weight as light polypeptide chain and as heavy polypeptide chain. The recognized immunoglobulin genes include the different constant region genes as well as the myriad immunoglobulin variable region genes. Immunoglobulins may exist in a variety of formats, including, for example, single heavy and light chains, Fv, Fab, and F(ab)$_2$ as well as single chains (scFv) (e.g. Huston, J.S., et al., Proc. Natl. Acad. Sci. USA 85 (1988) 5879-5883; Bird, R.E., et al., Science 242 (1988) 423-426; and, in general, Hood et al., Immunology, Benjamin N.Y., 2nd edition (1984) and Hunkapiller, T. and Hood, L., Nature 323 (1986) 15-16).

An immunoglobulin in general comprises two light polypeptide chains and two heavy polypeptide chains. Each of the heavy and light polypeptide chain contains a variable region (the amino terminal portion of the polypeptide chain) which contains a binding domain that is able to interact with an antigen. Each of the heavy and light polypeptide chain comprises a constant region (the carboxyl terminal portion of the polypeptide chain). The constant region of the heavy chain mediates the binding of the antibody i) to cells bearing a Fc gamma receptor (FcγR), such as phagocytic cells, or ii) to cells bearing the neonatal Fc receptor (FcRn) also known as Brambell receptor. The variable domain of an immunoglobulin's light or heavy chain in turn comprises different segments, i.e. four framework regions (FR) and three hypervariable regions (CDR).

An "immunoglobulin fragment" denotes a fragment of a complete immunoglobulin which has retained the ability to bind to the same antigen as the complete immunoglobulin. A "complete immunoglobulin" is an immunoglobulin consisting of two light polypeptide chains and two heavy polypeptide chains, each of them comprising a variable region and a constant region. An "immunoglobulin conjugate" denotes a conjugate of an immunoglobulin with a further non-immunoglobulin polypeptide. The binding of the antigen is not diminished by the conjugation to the further polypeptide.

"Transcription terminator" as denoted within this application is a DNA sequence of 50-750 base pairs in length which gives the RNA polymerase the signal for termination of the mRNA synthesis. Very efficient (strong) terminators at the 3' end of an expression cassette are advisable to prevent the RNA polymerase from reading through particularly when using strong promoters. Inefficient transcription terminators can lead to the formation of an operon-like mRNA which can be the reason for an undesired, e.g. plasmid-coded, gene expression.

The term "linker polypeptide" as used within this application denotes peptidic linker polypeptides of natural and/or synthetic origin. They comprise a linear amino acid chain wherein the 20 naturally occurring amino acids are the monomeric building blocks. The chain has a length of from 1 to 50 amino acids, preferred between 3 and 25 amino acids. The linker polypeptide may contain repetitive amino acid sequences or sequences of naturally occurring polypeptides, such as polypeptides with a hinge-function. The linker polypeptide has the function to ensure that a polypeptide conjugated to another polypeptide can retains its binding properties by allowing the peptide to fold correctly and to be presented properly. Preferably the linker polypeptide is a polypeptide designated to be rich in glycine, glutamine, and/or serine residues. These residues are arranged e.g. in a small repetitive units of up to five amino acids, such as GGGGS, QQQQG, or SSSSG. This small repetitive unit may be repeated for two to five times to form a multimeric unit. At the amino- and/or carboxy-terminal ends of the multimeric unit up to six additional arbitrary, naturally occurring amino acids may be added.

Other synthetic peptidic linkers are composed of a single amino acid, that is repeated between 10 to 20 times, such as e.g. serine in the linker SSSSSSSSSSSSSSS. At each of the amino- and/or carboxy-terminal end up to six additional arbitrary, naturally occurring amino acids may be present.

The term "amino acid" as used within this application denotes a group of carboxy $\alpha$-amino acids which can be encoded by a nucleic acid comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

An "antifusogenic peptide" is a peptide which inhibits events associated with membrane fusion or the membrane fusion event itself, including, among other things, the inhibition of infection of uninfected cells by a virus due to membrane fusion. These antifusogenic peptides are preferably linear peptides. For example, they can be derived from the gp41 ectodomain, e.g. such as DP107, or DP178. Examples of such peptides can be found in US 5,464,933, US 5,656,480, US 6,013,263, US 6,017,536, US 6,020,459, US 6,093,794, US 6,060,065, US 6,258,782, US 6,348,568, US 6,479,055, US 6,656,906, WO 1996/19495, WO 1996/40191, WO 1999/59615, WO 2000/69902, and WO 2005/067960. For example, the amino acid sequences of such peptides comprise SEQ ID NO: 1 to 10 of US 5,464,933; SEQ ID NO: 1 to 15 of US 5,656,480; SEQ ID NO: 1 to 10 and 16 to 83 of US 6,013,263; SEQ ID NO: 1 to 10, 20 to 83 and 139 to 149 of US 6,017,536; SEQ ID NO: 1 to 10, 17 to 83 and 210 to 214 of US 6,093,794; SEQ ID NO: 1 to 10, 16 to 83 and 210 to 211 of US 6,060,065; SEQ ID NO: 1286 and 1310 of US 6,258,782; SEQ ID NO: 1129, 1278-1309, 1311 and 1433 of US 6,348,568; SEQ ID NO: 1 to 10 and 210 to 238 of US 6,479,055; SEQ ID NO: 1 to 171, 173 to 216, 218 to 219, 222 to 228, 231, 233 to 366, 372 to 398, 400 to 456, 458 to 498, 500 to 570, 572 to 620, 622 to 651, 653 to 736, 739 to 785, 787 to 811, 813 to 815, 816 to 823, 825, 827 to 863, 865 to 875, 877 to 883, 885, 887 to 890, 892 to 981, 986 to 999, 1001 to 1003, 1006 to 1018, 1022 to 1024, 1026 to 1028, 1030 to 1032, 1037 to 1076, 1078 to 1079, 1082 to 1117, 1120 to 1176, 1179 to 1213, 1218 to 1223, 1227 to 1237, 1244 to 1245, 1256 to 1268, 1271 to 1275, 1277, 1345 to 1348, 1350 to 1362, 1364, 1366, 1368, 1370, 1372, 1374 to 1376, 1378 to 1379, 1381 to 1385, 1412 to 1417, 1421 to 1426, 1428 to 1430, 1432, 1439 to 1542, 1670 to 1682, 1684 to 1709, 1712 to 1719, 1721 to 1753, 1755 to 1757 of US 6,656,906; or SEQ ID NO: 5 to 95 of WO2005/067960. The antifusogenic peptide has an amino acid sequence comprising of from 5 to 100 amino acids, preferably of from 10 to 75 amino acids and more preferred of from 15 to 50 amino acids.

"CCR5" means human CCR5 as described, e.g., in Oppermann, M., Cell Signal. 16 (2004) 1201-1210, and SwissProt P51681. The term "anti-CCR5 antibody" denotes an antibody specifically binding to CCR5 and optionally inhibiting HIV fusion with a target cell. Binding can be tested in a cell based in vitro ELISA assay (CCR5 expressing CHO cells). Binding is found if the antibody causes an S/N (signal/noise) ratio of 5 or more, preferably 10 or more at an antibody concentration of 100 ng/ml. The term "inhibiting HIV fusion with a target cell" refers to inhibiting HIV fusion with a target cell measured in an assay comprising contacting said target cell (e.g. PBMC) with the virus in the presence of the antibody in a concentration effective to inhibit membrane fusion between the virus and said cell and measuring e.g. luciferase reporter gene activity or the HIV p24 antigen concentration. The term "membrane fusion" refers to fusion between a first cell coexpressing CCR5 and CD4 polypeptides and a second cell or virus expressing an HIV env protein. Membrane fusion is determined by genetically engineered cells and/or viruses by a reporter gene assay (e.g. by luciferase reporter gene assay).

Preferred anti-CCR5 antibodies are mentioned in US 2004/0043033, US 6,610,834, US 2003/0228306, US 2003/0195348, US 2003/0166870, US 2003/0166024, US 2003/0165988, US 2003/0152913, US 2003/0100058, US 2003/0099645, US 2003/0049251, US 2003/0044411, US 2003/0003440, US 6,528,625, US 2002/0147147, US 2002/0146415, US 2002/0106374, US 2002/0061834, US 2002/0048786, US 2001/0000241, EP 1 322 332, EP 1 263 791, EP 1 207 202, EP 1 161 456, EP 1 144 0 0 6 , W O 2003/072766, WO 2003/066830, WO 2003/033666, WO 2002/083172, WO 02/22077, WO 01/58916, WO 01/58915, WO 01/43779, WO 01/42308, and WO 2006/103100. Especially preferred anti-CCR5 antibodies are described in WO 2006/103100.

"CD4" means human CD4 as described, e.g., in Brady, R.L. and Barclay, A.N., Curr. Top. Microbiol. Immunol. 205 (1996) 1-18 and SwissProt P01730. The term "anti-CD4 antibody" denotes an antibody specifically binding to CD4 and preferably inhibiting HIV fusion with a target cell. Binding can be tested in a cell based in vitro ELISA assay (CD4 expressing CHO cells). Binding is found if the antibody in question causes an S/N (signal/noise) ratio of 5 or more, preferably 10 or more at an antibody concentration of 100 ng/ml. The term "inhibiting HIV fusion with a target cell" refers to inhibiting HIV fusion with a target cell measured in an assay comprising contacting said target cell (e.g. PBMC) with the virus in the presence of the antibody in question in a concentration effective to inhibit membrane fusion between the virus and said cell and measuring, e.g., luciferase reporter gene activity or the HIV p24 antigen concentration. The term "membrane fusion" refers to fusion between a first cell expressing CD4 polypeptides and a second cell or virus expressing an HIV env protein. Membrane fusion is determined by genetically engineered cells and/or viruses by a reporter gene assay (e.g. by luciferase reporter gene assay).

Preferred anti-CD4 antibodies are mentioned in e.g. Reimann, K.A., et al., Aids Res. Human Retrovir. 13 (1997) 933-943, EP 0 512 112, US 5,871,732, EP 0 840 618, EP 0 854 885, EP 1 266 965, US 2006/0051346, WO 97/46697, WO 01/43779, US 6,136,310, WO 91/009966. Especially preferred anti-CD4 antibodies are described in US 5,871,732, and Reimann, K.A., et al., Aids Res. Human Retrovir. 13 (1997) 933-943, and WO 91/009966. An especially preferred anti-CD4 antibody is characterized in that it is a non-immunosuppressive or non-depleting antibody when administered to humans and also does not block binding of HIV gp120 to human CD4.

"HIV-1" denotes human immunodeficiency virus type 1 gp 120. The term "neutralizing anti-HIV-1 antibody" denotes an antibody specifically binding to HIV-1 gp120 at more than one conformational epitope and neutralizing gp120 binding abilities, as well as optionally inhibiting HIV fusion with a cell. Exemplary "neutralizing anti-HIV-1 antibodies" are B12 and 4KG5. These monoclonal antibodies are directed against conformational epitopes located in the HIV gp120. The B12 antibody epitope, e.g., is predicted to consist of four peptide segments of gp120 (residues V254-T257, D368-F376, E381-Y384 and 1420-1424), which are located at the periphery of the CD4 binding site (Bublil, E.M., et al, FASEB J. 20 (2006) 1762-1774; Zwick, M.B., et al., J. Virol. 77 (2003) 6965-6978). The B12 antibody can be redesigned to avoid recognition of epitopes present in normal tissues.

The current invention reports a polypeptide conjugate, wherein the conjugate comprises

a) a first polypeptide selected from SEQ ID NO: 01,
b) a second polypeptide selected from the group of linear antifusogenic peptides.

The first polypeptide comprised in the polypeptide conjugate according to the invention is selected from SEQ ID NO: 01.

The first polypeptide is the A subunit of human complement factor C1q. The amino acid sequence of the A subunit of human complement factor C1q, which is hereinafter denoted as C1qA, is given in SEQ ID NO: 01. A fragment of C1qA denotes an amino acid sequence of at least 12 consecutive amino acid residues of SEQ ID NO: 01, of at least 15 consecutive amino acid residues of SEQ ID NO: 01, or of at least 18 consecutive amino acid residues of SEQ ID NO: 01. Exemplary fragments of SEQ ID NO: 01 comprise

| | |
|---|---|
| KGSPGNIKDQ PRPAFSA | (SEQ ID NO: 02), |
| KGSPGNIKDQ PRPAFSAI | (SEQ ID NO: 03), |
| GARGIPGIKG TKGSPGNIKD QPRPAFSAIR R | (SEQ ID NO: 04), |

GARGIPGIKG TKGSPGNIKD QPRPAFSAIR RNPPMGGNVV IFDTVITNQE EPYQNHSGRF VCTVPGYYYF TFQVLSQWEI CLSIVSSSRG QVRRSLGFCD TTNKGLFQVV SGGMVLQLQQ GDQVWVEKDP KKGHIYQGSE ADSVFSGFLI FPSA           (SEQ ID NO: 05), or

(continued)

KGDQGEPGPS GNPGKVGYPG PSGPLGARGI PGIKGTKGSP GNIKDQPRPA

FSAIRRNPPM GGNVVIFDTV ITNQEEPYQN HSGRFVCTVP GYYYFTFQVL

SQWEICLSIV SSSRGQVRRS LGFCDTTNKG LFQVVSGGMV LQLQQGDQVW

VEKDPKKGHI YQGSEADSVF SGFLIFPSA

(SEQ ID NO: 06)

SEQ ID NO: 06 denotes the globular head of C1qA. A fragment of the globular head of C1qA denotes an amino acid sequence of at least 12 consecutive amino acid residues of SEQ ID NO: 06, of at least 15 consecutive amino acid residues of SEQ ID NO: 06, or of at least 18 consecutive amino acid residues of SEQ ID NO: 06.

The second polypeptide is selected from the group of antifusogenic peptides. For example, antifusogenic peptides are derived from the HIV-1 gp41 protein (SEQ ID NO: 07). These antifusogenic peptides are preferably linear peptides. Exemplary antifusogenic peptides are DP107, DP178, C-34, N-36, T-20, T-651, T-1249, T-1357, T-1357 variant, T-2635, HIV-1 gp41 ectodomain variant single mutant: I568P, and HIV-1 gp41 ectodomain variant quadruple mutant: I568P, L550E, L566E, I580E. The amino acid sequences of some antifusogenic peptides are given in Table 1.

**Table 1**: Amino acid sequence of antifusogenic peptides.

| antifusogenic peptide | amino acid sequence | SEQ ID NO: |
|---|---|---|
| DP-107 | NNLLRAIEAQ QHLLQLTVWG IKQLQARILA VERYLKDQ | 08 |
| DP-178 | QQEKNEQDLL ALDKWASLWT WFDISHWLWY IKIFIMIV | 09 |
| C-34 | WMEWDREINN YTSLIHSLIE ESQNQQEKNE QELL | 10 |
| N-36 | SGIVQQQNNL LRAIEAQQHL LQLTVWGIKQ LQARIL | 11 |
| T-20 | YTSLIHSLIE ESQNQQEKNE QELLELDKWA SLWNWF | 12 |
| T-651 | MTWMEWDREI NNYTSLIHSL IEESQNQQEK NEQELL | 13 |
| T-1249 | WQEWEQKITA LLEQAQIQQE KNEYELQKLD KWASLWEWF | 14 |
| T-1357 | WQEWEQKITA LLEQAQIQQE KNEYELQKLD KWASLWEWF | 15 |

(continued)

| antifusogenic peptide | amino acid sequence | SEQ ID NO: |
|---|---|---|
| T-1357 variant | MRGSHHHHHH AIDVIEGRWQ EWEQKITALL EQAQIQQEKN EYELQKLDKW ASLWEWFG | 16 |
| T-2635 | TTWEAWDRAI AEYAARIEAL IRAAQEQQEK NEAALREL | 17 |
| HIV-1 gp41 ectodomain variant single mutant: I568P | VQARQLLSGI VQQQNNLLRA IEGQQHLLQL TVWGPKQLQA RILAVERYLK DQQLLGIWGC SGKLICTTAV PWNASWSNKS LEQIWNNMTW MEWDREINNY TSLIHSLIEE SQNQQEKNEQ ELL | 18 |
| HIV-1 gp41 ectodomain variant quadruple mutant: I568P, L550E, L566E, I580E | MGAASMTLTV QARQLLSGIV QQQNNELRAI EGQQHLEQLT VWGPKQLQAR ELAVERYLKD QQLLGIWGCS GKLICTTAVP WNASWSNKSL EQIWNNMTWM EWDREINNYT SLIHSLIEES QNQQEKNEQE LL | 19 |

The amino acid sequence and the numbering of the positions are as in the BH8 reference strain (Locus HIVH3BH8; HIV-1 isolate LAI/IIIB clone BH8 from France; Ratner, L. et al., Nature 313 (1985) 277-284). Further examples of such antifusogenic peptides can be found in US 5,464,933, US 5,656,480, US 6,013,263, US 6,017,536, US 6,020,459, US 6,093,794, US 6,060,065, US 6,258,782, US 6,348,568, US 6,479,055, US 6,656,906, WO 1996/19495, WO 1996/40191, WO 1999/59615, WO 2000/69902, and WO 2005/067960. The antifusogenic peptide has an amino acid sequence comprising of from 5 to 100 amino acids, of from 10 to 75 amino acids, preferably of from 15 to 50 amino acids. Especially preferred antifusogenic peptides are C-34, T-20, T-1249, T-1357, T-651, T-2635, N-36, (Root, M.J., et al., Curr. Pharm. Des. 10 (2004) 1805-1825), DP-107 (Wild, C., et al., Proc. Natl. Acad. Sci. USA 91 (1994) 12676-12680), DP-178, HIV-1 gp41 ectodomain variant single mutant: I568P, and/or HIV-1 gp41 ectodomain variant quadruple mutant: I568P, L550E, L566E, I580E.

The conjugate according to the invention comprises more than one polypeptide. Thus, a polypeptide forms the N-terminus of the conjugate and one polypeptide forms the C-terminus of the conjugate. The N- to C-terminal order of these polypeptides is arbitrary. This allows any of the conjugated polypeptides to be at the N-terminus and any of the conjugated polypeptides to be at the C-terminus with the proviso that each of the polypeptides is present only once in the conjugate. In one embodiment the conjugate according to the invention comprises the first and second polypeptide in an order selected from the following orders:

N-terminus - first polypeptide - second polypeptide - C-terminus, or
N-terminus - second polypeptide - first polypeptide - C-terminus.

The conjugate according to the invention may comprise a further additional polypeptide, a linker polypeptide. Thus, in one embodiment the conjugate according to the invention comprises between the first and second polypeptide a linker polypeptide. Preferred linker polypeptides are shown in Table 2.

**Table 2: Linker polypeptides**

| Linker polypeptide No. | Linker polypeptide amino acid sequence | SEQ ID NO: |
|---|---|---|
| 1 | LSLSPGK | 20 |
| 2 | LSPNRGEC | 21 |
| 3 | $[GQ_4]_3$ | 22 |
| 4 | $[GQ_4]_3G$ | 23 |
| 5 | $[GQ_4]_3GNN$ | 24 |
| 6 | $GGG[SG_4]_2SGG$ | 25 |
| 7 | $GGG[SG_4]_2SGN$ | 26 |
| 8 | $[SG_4]_3$ | 27 |
| 9 | $[SG_4]_3G$ | 28 |
| 10 | $G[SG_4]_3T$ | 29 |
| 11 | $[SG_4]_3GG$ | 30 |
| 12 | $[SG_4]_3GGT$ | 31 |
| 13 | $[SG_4]_3GGN$ | 32 |
| 14 | $[SG_4]_3GAS$ | 33 |
| 15 | $[SG_4]_5$ | 34 |
| 16 | $[SG_4]_5G$ | 35 |
| 17 | $[SG_4]_5GG$ | 36 |
| 18 | $[SG_4]_5GAS$ | 37 |
| 19 | $G(S)_{15}G$ | 38 |
| 20 | $G(S)_{15}GAS$ | 39 |
| 21 | G | - |
| 22 | N | - |
| 23 | GST | - |
| 24 | $[(G)_4S]_3GAS$ | 40 |
| 25 | $[(G)_4S]_3G$ | 41 |
| 26 | $[(G)_4S]_5G$ | 42 |
| 27 | $[(G)_4S]_3GG$ | 43 |
| 28 | $[(G)_4S]_5GG$ | 44 |
| 29 | LSLSGG | 45 |
| 30 | LSLSPGG | 46 |
| 31 | $[G_3S]_5$ | 47 |
| 32 | $[G_3S]_5GGG$ | 48 |

Especially preferred are the linker polypeptides $[GQ_4]_3GNN$ (SEQ ID NO: 24), LSLSPGK (SEQ ID NO: 20), LSPNRGEC (SEQ ID NO: 21), LSLSGG (SEQ ID NO: 45), LSLSPGG (SEQ ID NO: 46), $[G_3S]_5$, (SEQ ID NO: 47), and $[G_3S]_5GGG$, (SEQ ID NO: 48). All linker polypeptides can be encoded by a nucleic acid molecule and therefore can be recombinantly expressed. As the linker polypeptides are themselves peptides, the polypeptide, which is connected to the polypeptide linker, is connected via a peptide bond that is formed between two amino acids. Therefore, the polypeptide conjugate according to the invention can be produced recombinantly by protein expression.

The invention in another embodiment comprises a polypeptide conjugate in which beside the first and second polypeptide an additional third polypeptide is conjugated.

The first polypeptide is the A subunit of human complement factor C1q, as described above. The second polypeptide is a polypeptide selected from the group of linear antifusogenic peptides, as described above. The third polypeptide is an antigen-binding fragment of an anti-CCR5 antibody or an anti-CD4 antibody, or an anti-HIV-1 antibody.

The term "antigen-binding" as used within this application denotes an antibody or a fragment thereof, which is binding to its antigen. In case of an anti-CCR5 antibody the binding is to the CCR5-receptor, in case of an anti-CD4 antibody the binding is to the CD4-receptor, and in case of an anti-HIV-1 antibody the binding is to the HIV gp 120. The binding affinity given with a $K_D$-value is $10^{-5}$ mol/l or lower (e.g. $10^{-8}$ mol/l), with a $K_D$-value of $10^{-7}$ mol/l or lower, or with a $K_D$-value of $10^{-9}$ mol/l or lower. The binding affinity is determined with a standard binding assay, such as surface plasmon resonance technique (BIAcore®). This binding affinity value has not to be treated as an exact value; it is merely a point of reference. It is used to determine and/or select e.g. anti-CCR5 antibodies or fragments thereof showing the immunoglobulin-typical specific target binding for the CCR5-receptor-antigen and, thus, haveing a therapeutic activity. This likewise applies also to anti-CD4 antibodies and anti-HIV-1 antibodies.

The term "group of antigen-binding fragments of anti-CCR5 antibodies" as used within this application denotes any fragment of an anti-CCR5 antibody, which has retained the ability of antigen-binding. Such fragments generally comprise at least a part of the variable domain of an anti-CCR5 antibody light or heavy chain. Such fragments may be, for example, single heavy or light chains; Fv-, Fab-, and F(ab)$_2$-fragments, as well as single chain antibodies (scFv).

Preferred anti-CCR5 antibodies, whose fragments are useful in the conjugates according to the invention, are expressed by hybridoma cell lines listed in Table 3, which have been deposited with Deutsche Sammlung von Mikroorganismen und Zellkulturen GmbH (DSMZ), Germany.

**Table 3: And-CCR5 antibody expressing hybridoma cell lines.**

| Cell line | Deposition No. | Date of Deposit |
|---|---|---|
| m<CCR5>Pz01.F3 | DSM ACC 2681 | 18.08.2004 |
| m<CCR5>Pz02.1C11 | DSM ACC 2682 | 18.08.2004 |
| m<CCR5>Pz03.1C5 | DSM ACC 2683 | 18.08.2004 |
| m<CCR5>Pz04.1F6 | DSM ACC 2684 | 18.08.2004 |

Preferred anti-HIV-1 antibodies, whose fragments are useful in the conjugates according to the invention, are B12 and 4KG5.

Preferred anti-CD4 antibodies are described in US 5,871,732, Reimann, K.A., et al., Aids Res. Human Retrovir. 13 (1997) 933-943, and WO 91/009966.

In the polypeptide conjugate comprising a first, second, and third polypeptide, six different N-terminal (N-terminus denoted as NH$_2$) to C-terminal (C-terminus denoted as COOH) orders are possible. This group of orders comprises

(1) NH$_2$ - first polypeptide - second polypeptide - third polypeptide - COOH,
(2) NH$_2$ - first polypeptide - third polypeptide - second polypeptide - COOH,
(3) NH$_2$ - second polypeptide - first polypeptide - third polypeptide - COOH,
(4) NH$_2$ -second polypeptide - third polypeptide - first polypeptide - COOH,
(5) NH$_2$ - third polypeptide - first polypeptide - second polypeptide - COOH,
(6) NH$_2$ - third polypeptide - second polypeptide - first polypeptide - COOH.

It is optionally possible to include linker polypeptides between each of the conjugated polypeptides. In this case, e.g., the order (1) including one linker polypeptide in addition comprises four different orders

(1) NH$_2$ - first polypeptide - second polypeptide - third polypeptide - COOH,
(1a) NH$_2$ - first polypeptide - linker polypeptide - second polypeptide - third polypeptide - COOH,
(1b) NH$_2$ - first polypeptide - second polypeptide - linker polypeptide - third polypeptide - COOH,
(1c) NH$_2$ - first polypeptide - linker polypeptide - second polypeptide - linker polypeptide - third polypeptide - COOH.

Thus, in the presence of all optional linker polypeptides twenty-four different orders are possible.

A polypeptide conjugate according to the invention comprises each polypeptide at maximum once with exception of the linker polypeptide, which can be comprised up to three times.

Therefore, the current invention comprises a polypeptide conjugate, which comprises

a) a first polypeptide (1st pp) selected from SEQ ID NO: 01,
b) a second polypeptide (2nd pp) selected from the group of linear antifusogenic peptides,
c) a third polypeptide (3rd pp) selected from the group of antigen-binding fragments of anti-CCR5 antibodies, or the group of antigen-binding fragments of anti-CD4 antibodies,
d) a linker polypeptide (link pp) connecting said first, second, and/or third polypeptide,

whereby the polypeptides have an N- to C-terminal order of

$$[\text{1st pp}]_a - [\text{link pp}]_m - [\text{2nd pp}]_b - [\text{link pp}]_n - [\text{3rd pp}]_c - [\text{link pp}]_o - [\text{1st pp}]_d - [\text{link pp}]_p - [\text{2nd pp}]_e - [\text{link pp}]_q - [\text{3rd pp}]_f - [\text{link pp}]_r - [\text{1st pp}]_g$$

wherein a, b, c, d, e, f, g, m, n, o, p, q, r are all an integer of a value of 0 or 1,

with $a + d + g = 1$,
$b + e = 1$,
$c + f = 0$ or 1,
m, n, o, p, q, r independently of each other are either 0 or 1,

with 0 denoting the absence and with 1 denoting the presence of the corresponding polypeptide at the specified position of said conjugate.

In a preferred embodiment the polypeptides have an N- to C-terminal order of

$$[\text{3rd pp}]_c - [\text{link pp}]_o - [\text{1st pp}]_d - [\text{link pp}]_p - [\text{2nd pp}]_e - [\text{link pp}]_q - [\text{1st pp}]_g,$$

wherein c, d, e, g, o, p, q are all an integer of a value of 0 or 1,

with $c = 1$
$d + g = 1$,
$e = 1$,
o, p, q are independently of each other either 0 or 1,

with 0 denoting the absence and with 1 denoting the presence of the corresponding polypeptide at the corresponding position of said conjugate.

The current invention also comprises a nucleic acid encoding a polypeptide conjugate according to the invention. Another aspect of the invention is a cell line comprising a nucleic acid encoding a polypeptide conjugate according to the invention.

The current invention also comprises a method for the production of a polypeptide conjugate according to the invention, comprising the steps of

a) cultivating a host cell comprising a nucleic acid encoding a polypeptide conjugate according to the invention under conditions suitable for the expression of the polypeptide conjugate, and
b) recovering the polypeptide conjugate from the cell or the culture medium.

The term "under conditions suitable for the expression of the polypeptide conjugate" denotes conditions which are used for the cultivation of a cell expressing a heterologous polypeptide and which are known to or can easily be determined by a person skilled in the art. It is also known to a person skilled in the art that these conditions may vary depending on the type of cell cultivated and type of polypeptide expressed. In general the cell is cultivated at a temperature, e.g. between 20°C and 40°C, and for a period of time sufficient to allow effective production of the polypeptide conjugate, e.g. for 4 to 28 days. In one embodiment is the host cell a mammalian cell.

The invention further comprises a pharmaceutical composition, containing a polypeptide conjugate according to the invention, or a pharmaceutically acceptable salt thereof together with a pharmaceutically acceptable excipient or carrier.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion. Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skilled in the art.

Actual dosage levels of the active ingredients in the pharmaceutical compositions of the present invention may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

The invention further comprises the use of a polypeptide conjugate according to the invention for the manufacture of a medicament for the treatment of viral infections. Preferably the viral infection is a HIV infection. The invention also comprises the use of a polypeptide conjugate according to the invention for the treatment of a patient in need of an antiviral treatment. The invention also comprises the use of a conjugate according to the invention for the treatment of a patient suffering from immunodeficiency syndromes such as AIDS.

The following examples, sequence listing and figures are provided to aid the understanding of the present invention, the true scope of which is set forth in the appended claims.

## Description of the Figures

**Figure 1**  Annotated plasmid map of pQE80_C1qA.
**Figure 2**  Annotated plasmid map of pQE80_Rob 1 (Fusion protein T1357-C1qA).
**Figure 3**  Annotated plasmid map of pQE80_Rob 2 (Fusion protein C1qA-T1357).
**Figure 4**  16% Tricine SDS-PAGE of purified protein a1) non reduced, a2) reduced (Lane 1 IB-Preparation Rob I, Lane 2 IB-Preparation Rob II, Lane 3 Wash Rob I, Lane 4 Wash Rob II, Lane 5 Biomass Rob I Lane 6 Biomass Rob II); b) Lanes 7, 8 and 9 IB-Preparation C1qA Reduced, Lanes 10 and 12 Biomass C1qA reduced, Lanes 13, 14 and 15 IB-Preparation C1qA Non-reduced)
**Figure 5**  Western blot with a peptide encompassing gp41 amino acid position 593-621

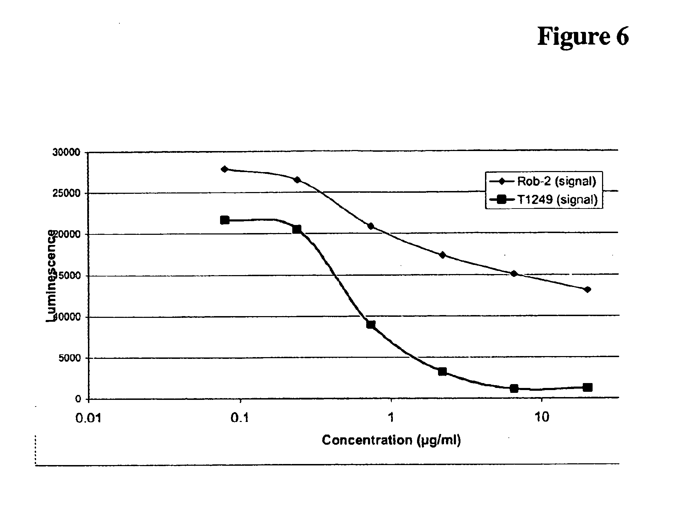

**Figure 6**  Analysis of the Activity of ROB II and T-1249 in fusion-inhibition Assays

## Example 1

### Materials & Methods

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered according to EU numbering (Edelman, G.M., et al., Proc. Natl. Acad. Sci. USA 63 (1969) 78-85; Kabat, E.A., et al., Sequences of Proteins of Immunological Interest, 5th ed., Public Health Service, National Institutes of Health, Bethesda, MD, (1991)).

### Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions.

## Gene synthesis

Desired gene segments were prepared from oligonucleotides made by chemical synthesis. The 100 - 600 bp long gene segments, which are flanked by singular restriction endonuclease cleavage sites, were assembled by annealing and ligation of oligonucleotides including PCR amplification and subsequently cloned via the Eco R1/HindIII restriction sites into the pQE80L vector (Qiagen, Hilden, Germany). The DNA sequence of the subcloned gene fragments were confirmed by DNA sequencing.

## Protein determination

The protein concentration of the conjugate was determined by determining the optical density (OD) at 280 nm, using the molar extinction coefficient calculated on the basis of the amino acid sequence.

## Example 2

## Construction of the expression plasmids

The T5 RNA polymerase-based bacterial pQE80 expression vector was purchased from Qiagen (Hilden, Germany)

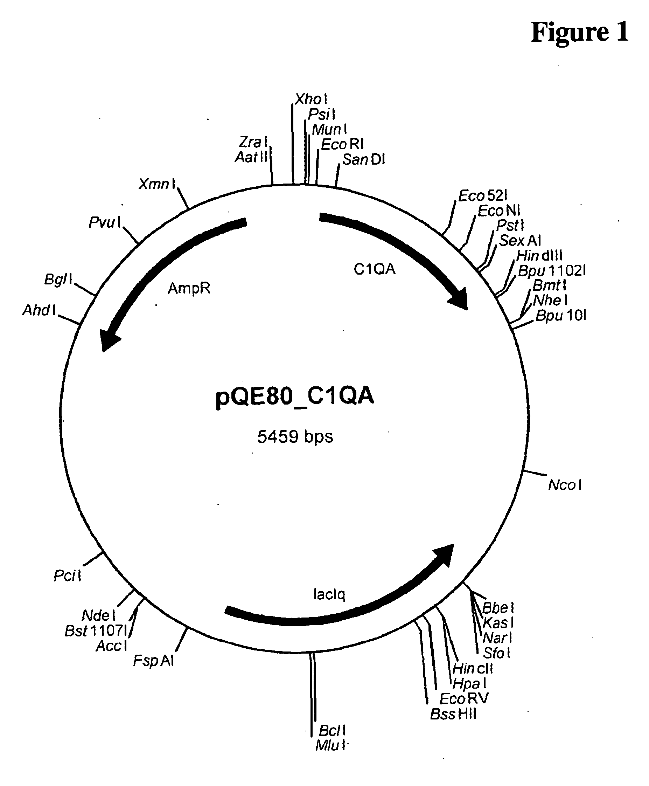

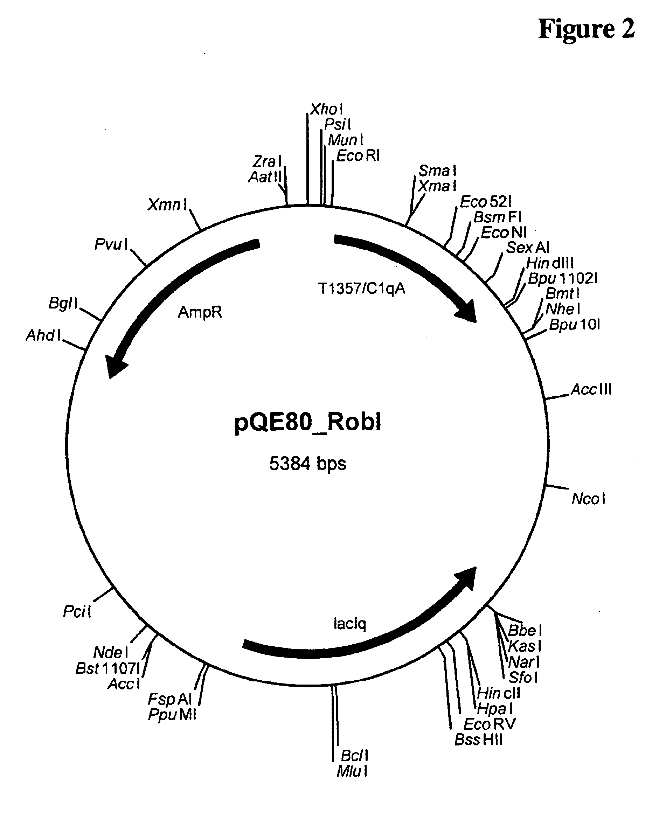

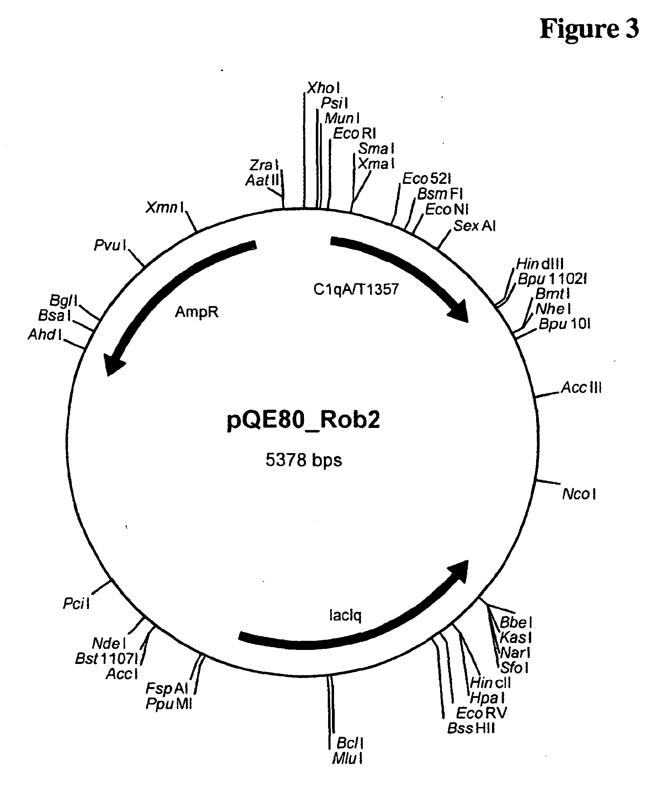

The EcoRI and HindIII restriction sites were used for the insertion of the nucleic acid sequence encoding C1qA (SEQ ID NO: 6) to generate expression plasmid pQE80_C1qA (for annotated plasmid map see Figure 1). The EcoRI and HindIII restriction sites was used for the insertion of the sequence encoding the conjugate comprising in N- to C-terminal direction the antifusogenic peptide T-1357 and the C1qA (SEQ ID NO: 49, 50) to generate expression plasmid pQE80_Rob I (for annotated plasmid map see Figure 2). The EcoRI and HindIII restriction sites was used for the insertion of the sequence encoding the conjugate comprising in N- to C-terminal direction the C1qA and T-1357 (SEQ ID NO: 51) to generate expression plasmid pQE80_Rob 2 (for annotate plasmid map see Figure 3)

## Example 3

## Production and purification of the polypeptide conjugates

E.coli cells were transformed with the expression plasmids obtained in Example 2. The transformed bacteria were selected by ampicillin resistance. Starting cultures with OD of 0.1 OD/mL were inoculated. Cultures were grown in SB-medium (32g peptone, 20 g yeast extract, 5 g NaCl and 5 mL 1 M NaOH ad 1L water) supplemented with 0.5 μg/ml ampicillin at 37 ˚C. The culturing was finished after reaching an OD600nm higher than 0.8-1.0. The culture broth was centrifuged and the cells in the pellet were disrupted with high pressure in a buffer containing 12.11 g/l TRIS-hydroxymethyl amino methane (TRIS), 1 mM MgSO$_4$, pH adjusted with 25 % (w/v) HCl to 7.0. Per 100 mg biomass 500 ml buffer were used. After disruption of the cells the suspension was centrifuged. The pellet was washed with a solution containing 200 ml/130 % (w/v) Brij, 1.5 M NaCl, and 60 mM EDTA, with the pH adjusted to 7.0. After a second centrifugation step the IBs were washed with 100 mM TRIS, 20 mM EDTA, pH 6.5. After a final centrifugation step the IBs were centrifuged and stored at -20 ˚C. Homogeneity of the sample was confirmed with SDS-PAGE. No additional purification steps were necessary.

The IBs were solubilized in 30 mM KOH at pH 11.5-12.0 by stirring for 30 min at room temperature. After complete solubilization of the samples, renaturation followed by pulsing the solubilisate in a 50 mM borate buffer solution, pH 8.5, to a maximal concentration of 0.3 mg/ml.

## Example 4

## Expression analysis using SDS PAGE

The renatured conjugates were processed by sodium dodecyl sulfate (SDS) polyacrylamide gel electrophoresis (SDS-PAGE) according to Schagger, H., and von Jagow, G., Anal Biochem. 166 (1997) 368-379.

## SDS-PAGE

LDS sample buffer, fourfold concentrate (4x): 4 g glycerol, 0.682 g TRIS-Base, 0.666 g TRIS-hydrochloride, 0.8 g LDS (lithium dodecyl sulfate), 0.006 g EDTA (ethylene diamin tetra acid), 0.75 ml of a 1% by weight (w/w) solution of Serva Blue G250 in water, 0.75 ml of a 1% by weight (w/w) solution of phenol red, add water to make a total volume of 10 ml.

The renatured polypeptide conjugate was centrifuged to remove debris. An aliquot of the clarified supernatant was admixed with 1/4 volumes (v/v) of 4xLDS sample buffer and 1/10 volume (v/v) of 0.5 M 1,4-dithiotreitol (DTT). Then the samples were incubated for 10 min. at 70 ˚C and protein separated by SDS-PAGE. The NuPAGE® Pre-Cast gel system (Invitrogen) was used according to the manufacturer's instruction. In particular, 10 % NuPAGE® Novex® Bis-TRIS Pre-Cast gels (pH 6.4) and a NuPAGE® MOPS running buffer was used.

## Example 5

### Construction of the expression plasmid

The T5 RNA polymerase-based bacterial pQE80 expression vector was purchased from Qiagen (Hilden, Germany)

The EcoRI and HindIII restriction sites are used for the insertion of the nucleic acid sequence encoding anti-CCR5 antibody light chain - C1qA - T-1249 conjugate to generate expression plasmid pQE80_Rob 3.

## Example 6

### Cell-Cell Fusion Assay

It is describe a cell line-based assay for the evaluation of human immunodeficiency virus type 1 (HIV-1) neutralization. The assay is based on CEM.NKR cells, transfected to express the HIV-1 coreceptor CCR5 to supplement the endogenous expression of CD4 and the CXCR4 coreceptor. The resulting CEM.NKR-CCR5 cells efficiently replicate primary HIV-1 isolates of both R5 and X4 phenotypes. A comparison of the CEM.NKR-CCR5 cells with mitogen-activated peripheral blood mononuclear cells (PBMC) in neutralization assays with sera from HIV-1-infected individuals or specific anti-HIV-1 monoclonal antibodies shows that the sensitivity of HIV-1 neutralization is similar in the two cell types.

At day 1, gp160-expressing HeLa cells ($2 \times 10^4$ cells / 50$\mu$l / well) are seeded in a white 96 microtiter plate in DMEM medium supplemented with 10% FCS and 2 $\mu$g/ml doxycycline. At day 2, 100$\mu$l of supernatant sample or antibody control per well is added in a clear 96 microtiter plate. Then 100 $\mu$l containing $8 \times 10^4$ CEM-NKr-Luc suspension cells in medium are added and incubated 30 min. at 37 ˚C. The HeLa cell culture medium is aspirated from the 96 well plate, 100 $\mu$l from the 200 $\mu$l antibody/CEM-NKr-Luc mixture is added and incubated over night at 37˚C. At day 3, 100 $\mu$l/well Bright-Glo™ Luciferase assay substrate (1,4-dithiothreitol and sodium dithionite; Promega Corp., USA) is added and luminescence is measured after a minimum of 15 min. incubation at RT.

### Materials

HeLa-R5-16 cells (cell line to express HIV gp160 upon doxycycline induction) are cultured in DMEM medium containing nutrients and 10 % FCS with 400 $\mu$g/ml G418 and 200 $\mu$g/ml Hygromycin B. CEM.NKR-CCR5-Luc (Catalog Number: 5198, a T-cell line available from NIH AIDS Research & Reference Reagent Program McKesson BioServices Corporation Germantown, MD 20874, USA). Cell Type: CEM.NKR-CCR5 (Cat. #4376) is transfected (electroporation) to express the luciferase gene under the transcriptional control of the HIV-2 LTR and propagated in RPMI 1640 containing 10% fetal bovine serum, 4 mM glutamine, penicillin/streptomycin (100 U/mL Penicillin, 100 $\mu$g/mL Streptomycin), and 0.8 mg/ml geniticin sulfate (G418). Growth Characteristics: Round lymphoid cells, morphology not very variable. Cells grow in suspension as single cells, which can form small clumps. Split 1:10 twice weekly. Special Characteristics: Express luciferase activity after transactivation of the HIV-2 LTR. Suitable for infection with primary HIV isolates, for neutralization and drug-sensitivity assays (Spenlehauer, C., et al., Virology 280 (2001) 292-300; Trkola, A., et al., J. Virol. 73 (1999) 8966-8974). The cell line was obtained through the NIH AIDS Research and Reference Reagent Program, NIAID, NIH from Drs. John Moore and Catherine Spenlehauer. Bright-Glo™ Luciferase assay buffer (Promega Corp. USA, Part No E2264B), Bright-Glo™, Luciferase assay substrate (Promega Corp. USA, part No EE26B).

## Example 7

### Determination of the binding affinity of polypeptides

Binding affinities of polypeptides based on the HR1-HR2 interaction of the HIV-1 gp41 protein (HR, Heptad Repeat 1 and 2 region) were measured by Surface Plasmon Resonance (SPR) using a BIAcore® 3000 instrument (Pharmacia, Uppsala, Sweden) at 25 ˚C.

The BIAcore® system is well established for the study of molecule interactions. It allows a continuous real-time monitoring of ligand/analyte bindings and thus the determination of association rate constants ($k_a$), dissociation rate constants ($k_d$), and equilibrium constants ($K_D$). SPR-technology is based on the measurement of the refractive index close to the surface of a gold coated biosensor chip. Changes in the refractive index indicate mass changes on the surface caused by the interaction of immobilized ligand with analyte injected in solution. If molecules bind immobilized ligand on the surface the mass increases, in case of dissociation the mass decreases.

**Binding Assay**

The Sensor Chip SA (SA, Streptavidin) was pre-washed by three consecutive 1-minute injections of 1 M NaCl in 50 mM NaOH. Then the biotinylated HR1 peptide Biotin-T-2324 (SEQ ID NO: 52) was immobilized on a SA-coated sensor chip. To avoid mass transfer limitations the lowest possible value (ca. 200 RU, Resonance Units) of HR1 peptide dissolved in HBS-P buffer (10 mM HEPES, pH 7.4, 150 mM NaCl, 0.005% (v/v) Surfactant P20) was loaded onto the SA-chip. Before the measurements were started the chip was regenerated a first time with a one minute pulse of 0.5% (w/v) sodium dodecyl sulfate (SDS) at a flow rate of 50 $\mu$L/min.

The polypeptide conjugates to be analyzed were first dissolved in 50 mM NaHCO$_3$, pH 9, at a concentration of about 1 mg/mL and then diluted in HPS-P buffer to various concentrations ranging from 25 to 1.95 nM. The sample contact time was 5 min. (association phase). Thereafter the chip surface was washed with HBS-P for 5 min. (dissociation phase). All interactions were performed at exactly 25˚C (standard temperature). During a measurement cycle the samples were stored at 12˚C. Signals were detected at a detection rate of one signal per second. Samples were injected at increasing concentrations at a flow rate of 50 $\mu$L/min over the HR1 coupled biosensor element. The surface was regenerated by 1 min. washing with 0.5% (w/v) SDS solution at a flow rate of 50 $\mu$L/min.

The equilibrium constants ($K_D$), defined as $k_a/k_d$ were determined by analyzing the sensorgram curves obtained with several different concentrations, using BIAevaluation 4.1 software package. Non specific binding was corrected by subtracting the response value of a HR2 containing polypeptide interaction with the free Streptavidin surface from the value of the HR2-HR1 interaction. The fitting of the data followed the 1:1 Langmuir binding model.

**Table 5: BIAcore® analysis of the binding to the HR1 region**

| sample name | $k_a$ [1/ms] | $k_d$ [1/s] | $K_A$ [1/M] | $K_D$ [M] |
|---|---|---|---|---|
| T-1249 | $6.4 \times 10^5$ | $8.45 \times 10^{-4}$ | $7.56 \times 10^8$ | $1.32 \times 10^{-9}$ |
| Rob 1 | $8.8 \times 10^4$ | $4.76 \times 10^{-4}$ | $1.85 \times 10^8$ | $5.41 \times 10^{-9}$ |
| Rob 2 | $3.5 \times 10^4$ | $3.11 \times 10^{-5}$ | $1.13 \times 10^9$ | $8.88 \times 10^{-10}$ |

**Example 8**

**Western Blot Analysis**

The following samples (15 $\mu$l each = 1-5 $\mu$g) were transferred to a 10 % Bis-TRIS NuPAGE-Gel under reducing conditions:

Lane 1 - multimarker
Lane 2 - borate buffer
Lane 3 - T-1249 (approx. 5 kDa)
Lane 4 - Rob I (26 kDa)
Lane 5 - Rob II (25 kDa)
Lane 6 - C1qA (28 kDa)
Lane 7 - empty
Lane 8 - magic Mark

Transfer buffer: 192 mM glycine, 25 mM TRIS, 20 % methanol (v/v).
After SDS-PAGE the separated conjugates were transferred electrophoretically (25 V, 1 hour) to a PVDF filter membrane (pore size: 0.45 $\mu$m, Invitrogen Corp.) according to the "Semidry-Blotting-Method" of Burnette (Burnette, W.N., Anal. Biochem. 112 (1981) 195-203).
After the blot was the membrane immersed in 5 % membrane blocking agent (Amersham Biosciences) in TBST (1110 mM TRIS buffer, supplemented with 150 mM NaCl, 1 ml Tween® 20 adjusted pH 7.5) for approx. 30 min. at room temperature with shaking and afterwards at 4 ˚C over night. After the blocking step is the membrane washed fro three times with TBST.

For detection the blocked membrane was incubated with the biotinylated peptide HIV-gp41P2(593-621)-Bi with shaking for 3 hours with 5 $\mu$g/ml TBST, 0.15 mM $CaCl_2$ and 12 mM $MgCl_2$.

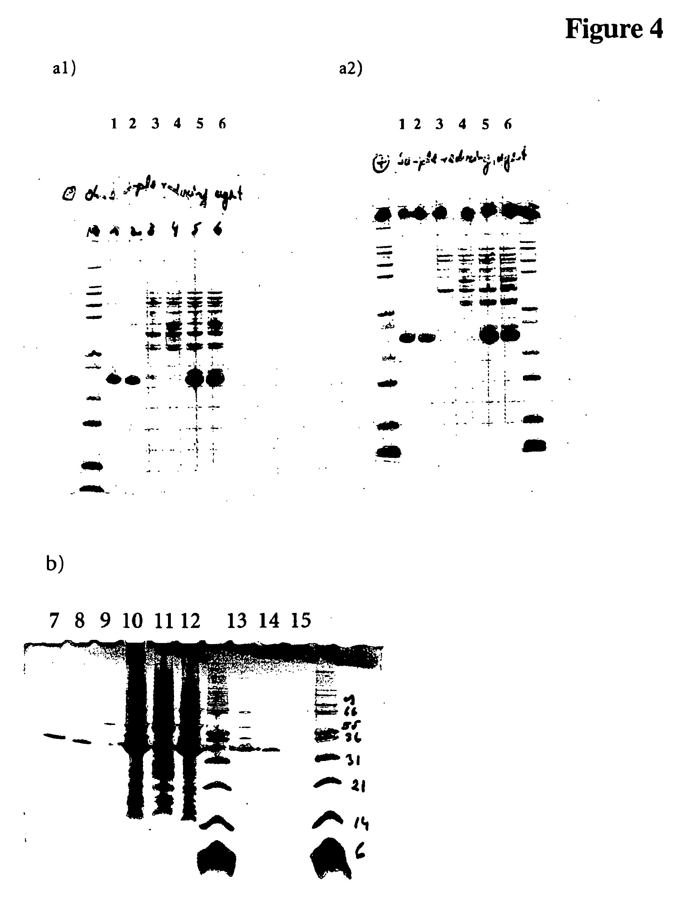

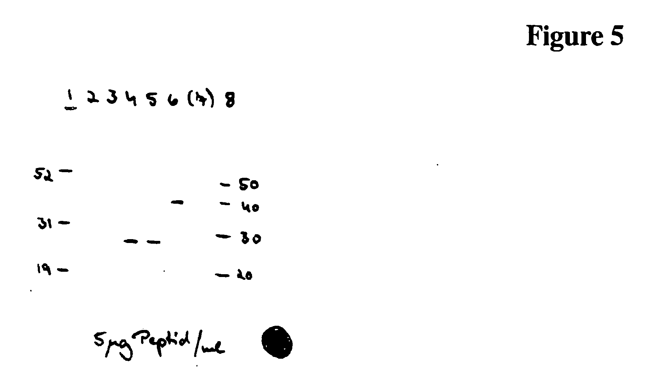

After the developing step was the membrane washed with TBST and incubated with Lumi-Light[PLUS] Western-Blotting Substrate and afterwards developed (see Figure 4 for SDS gel and Figure 5 for Western Blot).

**Claims**

1.  A polypeptide conjugate, **characterized in that** said conjugate comprises

    a) a first polypeptide selected from the A subunit of human complement factor C1q of SEQ ID NO: 01,
    b) a second polypeptide selected from the group of linear antifusogenic peptides which inhibit events associated with membrane fusion or the membrane fusion event itself, including, among other things, the inhibition of infection of uninfected cells by a virus due to membrane fusion.

2.  A conjugate according to claim 1, **characterized in that** said first and said second polypeptide have an order selected from the following group of orders:

    N-terminus - first polypeptide - second polypeptide - C-terminus, or
    N-terminus - second polypeptide - first polypeptide - C-terminus.

3.  A conjugate according to any one of the preceding claims, **characterized in that** said conjugate comprises between said first and said second polypeptide a linker polypeptide.

4.  A polypeptide conjugate, **characterized in that** said conjugate comprises

    a) a first polypeptide (1st pp) selected from the A subunit of human complement factor C1q of SEQ ID NO: 01, and
    b) a second polypeptide (2nd pp) selected from the group of linear antifusogenic peptides which inhibit events associated with membrane fusion or the membrane fusion event itself, including, among other things, the inhibition of infection of uninfected cells by a virus due to membrane fusion, and
    c) a third polypeptide (3rd pp) selected from the group of antigen-binding fragments of anti-CCR5 antibodies, or antigen-binding fragments of anti-HIV-1 antibodies, or the group of antigen-binding fragments of anti-CD4 antibodies, and
    d) a linker polypeptide (link pp) connecting said first, second, and/or third polypeptide,

    whereby the polypeptides of said polypeptide conjugate have an N- to C-terminal order of

$$[1st\ pp]a - [link\ pp]m - [2nd\ pp]b - [link\ pp]n - [3rd\ pp]c - [link\ pp]o -$$
$$[1st\ pp]d - [link\ pp]p - [2nd\ pp]e - [link\ pp]q - [3rd\ pp]f - [link\ pp]r -$$
$$[1st\ pp]g$$

wherein a, b, c, d, e, f, g, m, n, o, p, q, r are all an integer of a value of 0 or 1,
with

$a + d + g = 1$,
$b + e = 1$,
$c + f = 0$ or $1$,
m, n, o, p, q, r are independently of each other either 0 or 1,

with 0 denoting the absence and with 1 denoting the presence of the corresponding polypeptide at the corresponding position of said conjugate.

5. A conjugate according to claims 3 or 4, **characterized in that** said linker polypeptide is selected from the group of polypeptide comprising SEQ ID NO: 20 to SEQ ID NO: 48.

6. A conjugate according to any one of the preceding claims, **characterized in that** said second polypeptide is selected from the group of antifusogenic peptides comprising SEQ ID NO: 08 to SEQ ID NO: 19.

7. A method for the production of a polypeptide conjugate according to claim 1 or 4, **characterized in that** the method comprises

a) cultivating a host cell comprising a nucleic acid encoding a polypeptide conjugate according to claim 1 or 4 under conditions suitable for the expression of the polypeptide conjugate, and
b) recovering the polypeptide conjugate from the cell or the culture medium.

8. A pharmaceutical composition, containing a polypeptide conjugate according to any one of claims 1 to 6, or a pharmaceutically acceptable salt thereof together with a pharmaceutically acceptable excipient or carrier.

9. Use of a polypeptide conjugate according to any one of claims 1 to 6 for the manufacture of a medicament for the treatment of a viral infection.

10. Use according to claim 9, **characterized in that** said viral infection is a HIV infection.

**Patentansprüche**

1. Polypeptid-Konjugat, **dadurch gekennzeichnet, dass** das Konjugat

a) ein erstes Polypeptid, ausgewählt aus der A-Untereinheit des humanen Komplementfaktors C1q der SEQ ID NO: 01,
b) ein zweites Polypeptid, ausgewählt aus der Gruppe der linearen antifusiogenen Peptide, die Ereignisse, die mit einer Membranfusion assoziiert sind oder das Membranfusions-Ereignis selbst inhibieren, einschließlich unter anderem die Inhibition der Infektion nicht infizierter Zellen durch ein Virus aufgrund einer Membranfusion,

umfasst.

2. Konjugat nach Anspruch 1, **dadurch gekennzeichnet, dass** das erste und zweite Polypeptid eine Anordnung, ausgewählt aus der folgenden Gruppe von Anordnungen aufweisen:

N-Terminus - erstes Polypeptid - zweites Polypeptid - C-Terminus oder
N-Terminus - zweites Polypeptid - erstes Polypeptid - C-Terminus.

3. Konjugat nach einem der vorherigen Ansprüche, **dadurch gekennzeichnet, dass** das Konjugat zwischen dem ersten und dem zweiten Polypeptid ein Linker-Polypeptid umfasst.

4. Polypeptid-Konjugat, **dadurch gekennzeichnet, dass** das Konjugat

a) ein erstes Polypeptid (1. PP), ausgewählt aus der A-Untereinheit des humanen Komplementfaktors G1q der SEQ ID NO: 01, und
b) ein zweites Polypeptid (2. PP), ausgewählt aus der Gruppe der linearen antifusiogenen Peptide, die Ereignisse, die mit einer Membranfusion assoziiert sind oder das Membranfusions-Ereignis selbst inhibieren, einschließlich unter anderem die Inhibition der Infektion nicht infizierter Zellen durch ein Virus aufgrund einer Membranfusion, und
c) ein drittes Polypeptid (3. PP), ausgewählt aus der Gruppe von Antigen-bindenden Fragmenten von anti-CCR5 Antikörpern oder Antigen-bindenden Fragmenten von anti-HIV-1 Antikörpern oder der Gruppe von Antigen-bindenden Fragmenten von anti-CD4 Antikörpern, und
d) ein Linker-Polypeptid (link PP), welches das erste, zweite und/oder dritte Polypeptid verbindet,

umfasst,
wobei die Polypeptide des Polypeptid-Konjugats eine N- zu C-terminale Anordnung wie folgt aufweisen

$$[1.\ PP]a - [link\ PP]m - [2.\ PP]b - [link\ PP]n - [3.\ PP]c - [link\ PP]o -$$
$$[1.\ PP]d - [link\ PP]p - [2.\ PP]e - [link\ PP]q - [3.\ PP]f - [link\ PP]r -$$
$$[1.\ PP]g$$

wobei a, b, c, d, e, f, g, m, n, o, p, q, r alle eine ganze Zahl eines Wertes von 0 oder 1 sind,
wobei
a + d + g = 1,
b + e = 1,
c + f = 0 oder 1,
m, n, o, p, q, r alle unabhängig voneinander entweder 0 oder 1 sind,

wobei 0 die Abwesenheit kennzeichnet und wobei 1 die Anwesenheit des korrespondierenden Polypeptide an der korrespondierenden Position des Konjugats kennzeichnet.

5. Konjugat nach Ansprüchen 3 oder 4, **dadurch gekennzeichnet, dass** das Linker-Polypeptid ausgewählt ist aus der Gruppe von Polypeptiden, umfassend SEQ ID NO: 20 bis SEQ ID NO: 48.

6. Konjugat nach einem der vorherigen Ansprüche, **dadurch gekennzeichnet, dass** das zweite Polypeptid ausgewählt ist aus der Gruppe der antifusiogenen Peptide, umfassend SEQ ID NO: 08 bis SEQ ID NO: 19.

7. Verfahren zur Herstellung eines Polypeptid-Konjugats nach Anspruch 1 oder 4, **dadurch gekennzeichnet, dass** das Verfahren

a) Kultivieren einer Wirtszelle, umfassend eine Nukleinsäure, die ein Polypeptid-Konjugat nach Anspruch 1 oder 4 kodiert, unter Bedingungen, die für die Expression des Polypeptid-Konjugats geeignet sind, und
b) Rückgewinnen des Polypeptid-Konjugats aus der Zelle oder dem Kulturmedium,

umfasst.

8. Pharmazeutische Zusammensetzung, enthaltend ein Polypeptid-Konjugat nach einem der Ansprüche 1 bis 6, oder ein pharmazeutisch akzeptables Salz davon zusammen mit einem pharmazeutisch akzeptablen Hilfsstoff oder Träger.

9. Verwendung eines Polypeptid-Konjugats nach einem der Ansprüche 1 bis 6 für die Herstellung eines Medikaments zur Behandlung einer viralen Infektion.

10. Verwendung nach Anspruch 9, **dadurch gekennzeichnet, dass** die virale Infektion eine HIV-Infektion ist.

**Revendications**

1. Conjugué polypeptidique, **caractérisé en ce que** ledit conjugué comprend

 a) un premier polypeptide choisi dans la sous-unité A du facteur C1q du complément humain de SEQ ID NO: 01,
 b) un deuxième polypeptide choisi dans le groupe des peptides antifusogènes linéaires qui inhibent les événements associés à la fusion membranaire ou l'événement de fusion membranaire lui-même, comprenant, entre autres, l'inhibition de l'infection de cellules non infectées par un virus due à la fusion membranaire.

2. Conjugué selon la revendication 1, **caractérisé en ce que** lesdits premier et deuxième polypeptides ont un ordre choisi dans le groupe d'ordres suivant:

 extrémité N-terminale - premier polypeptide - deuxième polypeptide - extrémité C-terminale, ou
 extrémité N-terminale - deuxième polypeptide - premier polypeptide - extrémité C-terminale.

3. Conjugué selon l'une quelconque des revendications précédentes, **caractérisé en ce que** ledit conjugué comprend un polypeptide lieur entre lesdits premier et deuxième polypeptides.

4. Conjugué polypeptidique, **caractérisé en ce que** ledit conjugué comprend

 a) un premier polypeptide (1er pp) choisi dans la sous-unité A du facteur C1q du complément humain de SEQ ID NO: 01, et
 b) un deuxième polypeptide (2ème pp) choisi dans le groupe des peptides antifusogènes linéaires qui inhibent les événements associés à la fusion membranaire ou l'événement de fusion membranaire lui-même, comprenant, entre autres, l'inhibition de l'infection de cellules non infectées par un virus due à la fusion membranaire, et
 c) un troisième polypeptide (3ème pp) choisi dans le groupe des fragments d'anticorps anti-CCR5 se liant à l'antigène, ou des fragments d'anticorps anti-VIH-1 se liant à l'antigène, ou le groupe des fragments d'anticorps anti-CD4 se liant à l'antigène, et
 d) un polypeptide lieur (pp lieur) reliant lesdits premier, deuxième et/ou troisième polypeptides,

 les polypeptides dudit conjugué polypeptidique ayant de l'extrémité N-terminale à l'extrémité C-terminale l'ordre suivant:

 $$[\text{1er pp}]a - [\text{pp lieur}]m - [\text{2ème pp}]b - [\text{pp lieur}]n - [\text{3ème pp}]c - [\text{pp lieur}]o - [\text{1er pp}]d - [\text{pp lieur}]p - [\text{2ème pp}]e - [\text{pp lieur}]q - [\text{3ème pp}]f - [\text{pp lieur}]r - [\text{1er pp}]g$$

 où $a$, $b$, $c$, $d$, $e$, $f$, $g$, $m$, $n$, $o$, $p$, $q$, $r$ sont tous des entiers d'une valeur de 0 ou 1, avec $a + d + g = 1$;
 $b + e = 1$,
 $c + f = 0$ ou $1$,
 $m$, $n$, $o$, $p$, $q$, $r$ sont indépendamment les uns des autres 0 ou 1,
 0 indiquant l'absence et 1 indiquant la présence du polypeptide correspondant à la position correspondante dudit conjugué.

5. Conjugué selon les revendications 3 ou 4, **caractérisé en ce que** ledit polypeptide lieur est choisi dans le groupe de polypeptides comprenant SEQ ID NO: 20 à SEQ ID NO: 48.

6. Conjugué selon l'une quelconque des revendications précédentes, **caractérisé en ce que** ledit deuxième polypeptide est choisi dans le groupe de peptides antifusogènes comprenant SEQ ID NO: 08 à SEQ ID NO: 19.

7. Procédé de production d'un conjugué polypeptidique selon la revendication 1 ou 4, **caractérisé en ce que** le procédé comprend

 a) la culture d'une cellule hôte comprenant un acide nucléique codant pour un conjugué polypeptidique selon la revendication 1 ou 4 dans des conditions appropriées pour l'expression du conjugué polypeptidique, et
 b) la récupération du conjugué polypeptidique à partir de la cellule ou du milieu de culture.

8. Composition pharmaceutique contenant un conjugué polypeptidique selon l'une quelconque des revendications 1 à 6, ou un de ses sels pharmaceutiquement acceptables, avec un excipient ou un support pharmaceutiquement acceptable.

9. Utilisation d'un conjugué polypeptidique selon l'une quelconque des revendications 1 à 6 pour la fabrication d'un médicament destiné au traitement d'une infection virale.

10. Utilisation selon la revendication 9, **caractérisée en ce que** ladite infection virale est une infection par le VIH.

# Figure 1

pQE80_C1QA

5459 bps

**Figure 2**

Xho I
Psi I
Mun I
Eco RI
Zra I
Aat II
Sma I
Xma I
Xmn I
Eco 52I
Bsm FI
Eco NI
Pvu I
Sex AI
Hin dIII
Bpu 1102I
Bgl I
Bmt I
Ahd I
Nhe I
Bpu 10I
AmpR
T1357/C1qA
Acc III
pQE80_Robl
5384 bps
Nco I
Pci I
laclq
Bbe I
Kas I
Nde I
Nar I
Bst 1107I
Sfo I
Acc I
Hin cII
Fsp AI
Hpa I
Ppu MI
Eco RV
Bss HII
Bcl I
Mlu I

**Figure 3**

# Figure 4

**Figure 5**

# Figure 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5464933 A
- US 5656480 A
- US 6258782 B
- US 6348568 B
- US 6656906 B
- US 6479055 B
- WO 02103026 A
- WO 9208796 A
- WO 9428143 A
- US 5888809 A
- US 6013263 A
- US 6017536 A
- US 6020459 A
- US 6093794 A
- US 6060065 A
- WO 199619495 A
- WO 199640191 A
- WO 199959615 A
- WO 200069902 A
- WO 2005067960 A
- US 20040043033 A
- US 6610834 B
- US 20030228306 A
- US 20030195348 A
- US 20030166870 A
- US 20030166024 A
- US 20030165988 A
- US 20030152913 A
- US 20030100058 A
- US 20030099645 A
- US 20030049251 A
- US 20030044411 A
- US 20030003440 A

- US 6528625 B
- US 20020147147 A
- US 20020146415 A
- US 20020106374 A
- US 20020061834 A
- US 20020048786 A
- US 20010000241 A
- EP 1322332 A
- EP 1263791 A
- EP 1207202 A
- EP 1161456 A
- EP 1144006 A
- WO 2003072766 A
- WO 2003066830 A
- WO 2003033666 A
- WO 2002083172 A
- WO 0222077 A
- WO 0158916 A
- WO 0158915 A
- WO 0143779 A
- WO 0142308 A
- WO 2006103100 A
- EP 0512112 A
- US 5871732 A
- EP 0840618 A
- EP 0854885 A
- EP 1266965 A
- US 20060051346 A
- WO 9746697 A
- US 6136310 A
- WO 91009966 A
- EP 07005096 A

**Non-patent literature cited in the description**

- **Shu, W. et al.** *Biochemistry,* 1999, vol. 38, 5378-5385
- **Sia, S.K. et al.** *Proc. Natl. Acad. Sci. USA,* 2002, vol. 99, 14664-14669
- **Otaka, A. et al.** *Angew. Chem. Int. Ed.,* 2002, vol. 41, 2937-2940
- **Sellar, G.C. et al.** *Biochem. J.,* 1991, vol. 274, 481-490
- **Moir, S. et al.** *J. Exp. Med.,* 2000, vol. 192, 637-646
- **Ratner, L. et al.** *Nature,* 1985, vol. 313, 277-284

- **Ebenbichler, C.F.** *J. Exp. Med.,* 1991, vol. 174, 1417-1424
- **Thielens, N.M. et al.** *J. Immunol.,* 1993, vol. 151, 6583-6592
- **Root, M.J. et al.** *Curr. Pharm. Des.,* 2004, vol. 10, 1805-1825
- Current Protocols in Molecular Biology. 1997, vol. I-II
- **Sambrook et al.** Molecular Cloning: A Laboratory Manual. Cold Spring Harbor Laboratory Press, 1989

- **Sambrook, J. et al.** Molecular Cloning: A laboratory manual. Cold Spring Harbor Laboratory Press, 1999
- **McGehee, R.E. et al.** *Mol. Endocrinol.,* 1993, vol. 7, 551-560
- **Treisman, R.** *Seminars in Cancer Biol.,* 1990, vol. 1, 47-58
- **O'Reilly, M.A. et al.** *J. Biol. Chem.,* 1992, vol. 267, 19938-19943
- **Ye, J. et al.** *J. Biol. Chem.,* 1994, vol. 269, 25728
- **Loeken, M.R.** *Gene Expr.,* 1993, vol. 3, 253-264
- Molecular Biology of the Gene. Benjamin/Cummings Publishing Company, Inc, 1987
- **Lemaigre, F.P. ; Rousseau, G.G.** *Biochem. J.,* 1994, vol. 303, 1-14
- **Gossen, M. ; Bujard, H.** *PNAS,* 1992, vol. 89, 5547-5551
- **Gossen, M. et al.** *Curr. Opin. Biotech.,* 1994, vol. 5, 516-520
- **Lusky, M. et al.** *Mol. Cell Bio.,* 1983, vol. 3, 1108-1122
- **Banerji, J. et al.** *Cell,* 1983, vol. 33, 729-740
- **Osborne, T.F. et al.** *Mol. Cell Bio.,* 1984, vol. 4, 1293-1305
- **Bendig, M.M.** Genetic Engineering. Academic Press, 1988, vol. 7, 91-127
- **Pelletier, J. et al.** *Nature,* 1988, vol. 334, 320-325
- **Jang, S.K. et al.** *J. Virol.,* 1989, vol. 63, 1651-1660
- **Davies, M.V. et al.** *J. Virol.,* 1992, vol. 66, 1924-1932
- **Adam, M.A. et al.** *J. Virol.,* 1991, vol. 65, 4985-4990
- **Morgan, R.A. et al.** *Nucl. Acids Res.,* 1992, vol. 20, 1293-1299
- **Sugimoto, Y et al.** *Biotechnology,* 1994, vol. 12, 694-698
- **Ramesh, N. et al.** *Nucl. Acids Res.,* 1996, vol. 24, 2697-2700
- **Mosser, D.D. et al.** *BioTechniques,* 1997, vol. 22, 150-161
- **Huston, J.S. et al.** *Proc. Natl. Acad. Sci. USA,* 1988, vol. 85, 5879-5883
- **Bird, R.E. et al.** *Science,* 1988, vol. 242, 423-426
- **Hood et al.** Immunology. 1984
- **Hunkapiller, T. ; Hood, L.** *Nature,* 1986, vol. 323, 15-16
- **Oppermann, M.** *Cell Signal,* 2004, vol. 16, 1201-1210
- **Brady, R.L. ; Barclay, A.N.** *Curr. Top. Microbiol. Immunol.,* 1996, vol. 205, 1-18
- **Reimann, K.A. et al.** *Aids Res. Human Retrovir,* 1997, vol. 13, 933-943
- **Bublil, E.M. et al.** *FASEB J.,* 2006, vol. 20, 1762-1774
- **Zwick, M.B. et al.** *J. Virol.,* 2003, vol. 77, 6965-6978
- **Wild, C. et al.** *Proc. Natl. Acad. Sci. USA,* 1994, vol. 91, 12676-12680
- **Kabat, E.A. et al.** Sequences of Proteins of Immunological Interest. Public Health Service, National Institutes of Health, 1991
- **Edelman, G.M. et al.** *Proc. Natl. Acad. Sci. USA,* 1969, vol. 63, 78-85
- **Sambrook, J. et al.** Molecular cloning: A laboratory manual. Cold Spring Harbor Laboratory Press, 1989
- **Schagger, H. ; Jagow, G.** *Anal Biochem.,* 1997, vol. 166, 368-379
- **Spenlehauer, C. et al.** *Virology,* 2001, vol. 280, 292-300
- **Trkola, A. et al.** *J. Virol.,* 1999, vol. 73, 8966-8974
- **Burnette, W.N.** *Anal. Biochem.,* 1981, vol. 112, 195-203